# EUROPEAN PATENT SPECIFICATION

(11) **EP 3 847 664 B1**
(45) Date of publication and mention of the grant of the patent: **25.11.2026**
(21) Application number: 18769952.5
(22) Date of filing: 05.09.2018
(51) Int. Cl.: G16H 50/20, G16H 20/10

(54) **NETWORK METHODS FOR NEURODEGENERATIVE DISEASES**
NETZWERKVERFAHREN FÜR NEURODEGENERATIVE ERKRANKUNGEN
PROCÉDÉS DE RÉSEAU POUR MALADIES NEURODÉGÉNÉRATIVES

(43) Date of publication of application: 14.07.2021
(73) Proprietor: Genting Taurx Diagnostic Centre SDN BHD, Kuala Lumpur 50250 (MY)
(72) Inventor: WISCHIK, Claude Michel, Aberdeen Aberdeenshire AB25 2ZD (GB); SCHELTER, Björn Olaf, Aberdeen Aberdeenshire AB24 3UE (GB); SOMMERLADE, Linda, Aberdeen Aberdeenshire AB24 5RP (GB); VUKSANOVIC, Vesna, Aberdeen Aberdeenshire AB25 2ZD (GB); STAFF, Roger Todd, Aberdeen Aberdeenshire AB25 2ZD (GB); ALLAN, Kevin, Aberdeen Aberdeenshire AB24 3FX (GB); MORSON, Suzannah Marie, Aberdeen, AB24 5RP (GB); TEE, Lip Jin, 70200 Seremban Negeri Sembilan (MY)
(74) Representative: Mewburn Ellis LLP
(86) International application number: PCT/EP2018/073906
(87) International publication number: WO 2020/048593

(56) References cited:
- WO-A1-2011/127609
- WO-A1-2011/127609
- WO-A1-2016/016833
- WO-A1-2017/172487
- WO-A2-2012/094621
- WO-A2-2014/153466
- US-A1- 2011 295 515
- US-A1- 2013 231 552
- US-A1- 2015 018 664

## Description

### Field of the Invention

The present invention relates to the application of network methods in investigating neurocognitive disorders.

### Background

Models of the human brain as a complex network of interconnected sub-units have improved the understanding of normal brain organisation, and have made it possible to address functional changes in neurological disorders. These sub-units constitute so called brain modules, i.e. groups of regions that have a high density of connections within them, and with a lower density of connections between groups. It has been suggested that the modular organisation of the brain underpins efficient integration between spatially segregated neural processes, which supports diverse cognitive and behavioural functions. Changes in brain networks can assist in identify patients with Alzheimer's disease (AD) and behavioural variant frontotemporal dementia (bvFTD).

For example, alterations in regional volumes have been identified in schizophrenia patients through study of structural networks in health and disease where pair-wise correlations of the cortical regional volume or thickness, as derived from in vivo measurements of T1-weighted magnetic resonance images (MRI), have been examined. This approach has shown clinical relevance by revealing alterations in regional volumes in schizophrenia patients. However, volumetric measures, which represent the product of cortical thickness (CT) and surface area (SA), may confound underlying differences. For example, consideration of changes in cortical thickness may provide insight into how disease alters the size, density, and arrangement of the cells within cortical layers. Changes in surface area, on the other hand, may provide information regarding disturbance in functional integration between groups of columns in diseased brains.

Previously, to monitor the effect of neuropharmacological interventions, whole brain or lobar volume has been used. However this is a relatively crude level of analysis.

As a further example of the use of networks in understanding the brain, as discussed in WO 2017/118733, EEG data collected from a patient can be used to detect the intensity and directionality of electrical flow within the brain.

A poster entitled "Organisation of cortical thickness networks in Alzheimer's disease and behavioural variant frontotemporal dementia across brain lobes" was presented by Vuksanovic et al. at the 6th Cambridge Neuroscience Symposium, Neural Networks in Health and Disease September 7-8 2017.

A further poster entitled " Divergent changes in structural correlation networks in Alzheimer's disease and behavioural variant frontotemporal dementia" was presented by Vuksanovic et al. at the ARUK Conference 2018, 20-21 March, London, UK.

A presentation entitled "Modular organisation of cortical thickness and surface area structural correlation networks in Alzheimer's disease (AD) and behavioural variant frontotemporal dementia (bvFTD)" was given by Vuksanovic, V, at the 10th SINAPSE Annual Scientific Meeting 25 June 2018 Edinburgh. WO 2011/127609 concerns brain-network-related analysis of encephalography data for monitoring brain function, classifying neurodegenerative disease states and assessing drug effects by comparing brain-derived signal characteristics before and after treatment.

### Summary

In a first aspect, the invention provides a method of determining patient response to a neuropharmacological intervention according to claim 1, a system configured to perform the method of the first aspect, and a computer program product comprising executable code stored on a non-transitory storage medium which, when run on a computer, causes the computer to perform the method of the first aspect.

Optional features of the invention will now be set out. These are applicable singly or in any combination with any aspect of the invention.

By correlation matrix, it may be meant that a structural correlation network is generated which may then be represented by a matrix.

In one embodiment, the patient response may be in the context of a clinical trial e.g. for assessing the efficacy of a pharmaceutical in the treatment of a neurocognitive disease. Thus the patient group (plurality of patients) may be treatment group who have been diagnosed with the disease, or maybe a control ('normal') group. Ultimately the efficacy of the pharmaceutical may be assessed in whole or in part based on the patient group response determined in accordance with the present invention, optionally compared with a comparator group who have not received the intervention.

The values for the cortical thickness and/or surface area may be averaged values obtained from the structural neurological data. The structured neurological data may be acquired from magnetic resonance imaging (MRI) data or computed tomography data for each patient. The structural neurological data and the further structural neurological data are obtained at different points in time. As discussed herein, the structural neurological data may be obtained via magnetic resonance imaging, computed tomography, or positron emission tomography for each patient. These techniques are well known *per se* to those skilled in the art - see e.g. Mangrum, Wells, et al. Duke Review of MRI Principles: Case Review Series E-book. Elsevier Health Sciences, 2018, and "Standardized low-resolution electromagnetic tomography (sLORETA): technical details" Methods Find Exp. Clin. Pharmacol. 2002:24 Suppl. D:5-12; Pascual-Marqui RD etc.

The plurality of cortical regions may be at least 60, or at least 65. For example, 68. The cortical regions may, for example, be those provided by the Desikan-Killiany Atlas (Desikan et al. 2006).

A p-value may be determined for each pair-wise correlation across a plurality of subjects, and may be compared to a significance level, wherein only p-values less than the significance level are used to generate the corresponding correlation matrix. In determining the pair-wise correlation between pairs of structure nodes, the corresponding values of each structure node may be compared to a reference value and their co-variance determined. The significance level may be referred to as alpha ('α').

In making the comparison, groups of structure nodes corresponding to a same lobe may be identified, and the comparison made between the first and second correlation matrix may utilize the same lobe.

Assigning the plurality of structure nodes corresponding to cortical regions of the brain may further include defining groups which contain structure nodes corresponding to homologous or non-homologous lobes. Comparing the first correlation matrix and the second correlation matrix includes comparing the number and/or density of correlations between different groups of structure nodes. Said another way, comparing the first and second correlation matrices may include comparing correlations between pairs of structure nodes which are non-homologous.

In some examples, comparing the first correlation matrix and the second correlation matrix may include comparing the number and/or density of correlations between groups of structure nodes located respectively in the frontal lobe (anterior nodes) and the parietal and occipital lobes (posterior nodes). It has been found that, in examples of efficacious neuropharmacological intervention, the number and/or density of inverse correlations between anterior and posterior nodes decreases. As inverse correlations are postulated to indicate compensatory link formulation whereby atrophy in one node is associated with hypertrophy in a functionally linked node, it will be appreciated that a decrease in the number and/or density of inverse correlations indicates a decrease in the number of compensatory links.

Typically, the neurocognitive disease or cognitive disorder is a neurodegenerative disorder causing dementia, for example a tauopathy.

The patients may have been diagnosed with a neurocognitive disease, for example Alzheimer's disease or behavioural-variant frontotemporal dementia. The disease may be mild or moderate Alzheimer's disease. The disease may be a mild cognitive impairment. However the findings of the present inventors described herein have applicability to other neurocognitive diseases also.

Diagnosis criteria and treatment of tauopathies, and other neurocognitive diseases, are known in the art and discussed, for example, in WO2018/019823, and references cited therein.

The disease may be behavioural variant frontotemporal dementia (bvFTD). Diagnostic criteria and treatment of bvFTD is discussed, for example, in WO 2018/041739, and references cited therein.

As explained herein, the topology of the disturbance in structural network is different in these two disease conditions (AD and bvFTD) and both are different from normal aging. The changes from normal are global in character, and are not restricted to fronto-temporal and temporo-parietal lobes respectively in bvFTD and AD and indicate an increase in both global correlation strength and in particular non-homologous inter-lobar connectivity defined by inverse correlations.

These changes appear to be adaptive in character, reflecting coordinated increases in cortical thickness and surface area that compensate for corresponding impairment in functionally linked nodes. The effects were more pronounced in the cortical thickness network in bvFTD and in the surface area network in AD.

The inventors have observed that an important change distinguishing both forms of dementia from normal elderly controls is the emergence of significant inverse correlation networks linking anterior and posterior brain regions which may relate to functional adaptations or compensations for impairment due to pathology. Specifically, inverse correlations are postulated to indicate compensatory link formation whereby atrophy in one node is associated with hypertrophy in a functionally linked node, it will be appreciated that a decrease in the number and/or density of inverse correlations indicates a decrease in the number of compensatory links.

Thus, if a neuropharmacological intervention is efficacious, it is expected that the network organisation will be brought back towards that observed with a normal (non-disease) comparator population. If the condition is treated at an early enough stage, the network organisation may be brought back to something entirely equivalent to the normal control. The method therefore provides an objective means of distinguishing disease-modifying treatments from symptomatic treatments: symptomatic treatments accentuate the abnormal network architecture, and may indeed accentuate the risk of transmission of (for example) prion-like disease processes to healthy brain regions. Conversely, disease-modifying drugs work in the opposite direction, reducing the need for compensatory input from relatively less impaired brain regions by normalising function in regions affected by pathology.

It will be appreciated, in the light of the disclosure herein, that analysis of structural or network organisation has particular utility in providing more power in clinical trials, thereby allowing for the use of fewer subjects and shorter treatment times. In particular, in diseases such as mild AD, mild cognitive impairment and pre-mild cognitive impairment etc., the clinical trial end-points (cognitive and function) can be relatively insensitive and so require large numbers of subjects and\or longer time periods (see WO2009/060191).

Thus, typically, the neuropharmacological intervention will be a pharmaceutical intervention.

The neuropharmacological intervention may be a symptomatic treatment. Such compounds include acetylcholinesterase inhibitors (AChEls) - these include tacrine, donepezil, rivastigmine, and galantamine. A further symptomatic treatment is memantine. These treatments are described in WO2018/041739.

As explained above, the inventors have found an increase in compensatory networks (number and/or density of non-homologous inverse correlations present in patient groups who have received such treatments).

The neuropharmacological intervention may be a disease modifying pharmaceutical rather than a symptomatic one. These treatments can be distinguished, for example, based on what happens when a patient is withdrawn from active treatment. Symptomatic agents defer the symptoms of the disease without affecting the fundamental disease process and do not change (or at least do not improve) the rate of longer term decline after an initial period of treatment. If, after withdrawal, the patient reverts to where they would have been without treatment, the treatment is deemed to be symptomatic (Cummings, J. L. (2006) Challenges to demonstrating disease-modifying effects in Alzheimer's disease clinical trials. Alzheimer's and Dementia, 2:263-271).

For example, a disease modifying treatment may be an inhibitor of pathological protein aggregation such as a 3,7-diaminophenothiazine (DAPTZ) compound. Such compounds are described in WO2018/041739, WO2007/110627, and WO2012/107706. The latter describes leuco-methylthioninium bis(hydromethanesulfonate) also known as leuco-methylthioninium mesylate (LMTM; USAN name: hydromethylthionine mesylate).

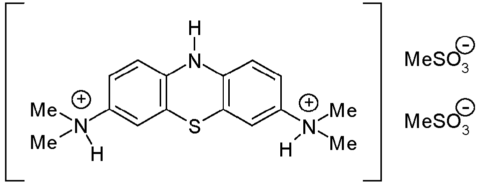

Treatment with LMTM has been shown to reduce compensatory network correlations (especially non-homologous, positive and inverse correlations).

The neuropharmacological intervention is a disease modifying pharmaceutical, and efficacy is established by reduction in number and/or density of correlations between anterior and posterior brain regions of the first correlation matrix and the second correlation matrix.

Thus it can be concluded that in examples of efficacious neuropharmacological intervention (for example a disease modifying treatment) the number and/or density of inverse correlations between anterior and posterior nodes decreases.

The invention may also be utilised for identifying functional adaptations or compensations for impairment due to pathology in a patient population, for example to investigate "cognitive reserve". The invention may be used in combination with conventional diagnostic or prognostic measures. These measures include the Alzheimer's Disease Assessment Scale - cognitive subscale (ADAS-Cog), National Institute of Neurological and Communicative Disorders and Stroke - Alzheimer's Disease and Related Disorders Association (NINCDS-ADRDA), Diagnostic and Statistical Manual of Mental Disorders, 4th Edn (DSMIV), and Clinical Dementia Rating (CDR) scale.

As explained above, the method of determining patient response to a neuropharmacological intervention may in turn be used to assess different patient cohorts in clinical trials of the neuropharmacological intervention. For example, the method may be for determining the effectiveness of a neuropharmacological intervention in a patient group. The method may be used for defining a patient group according to their patient response (e.g. in terms of the correlations/inverse correlations determined). The patient group may be identified in relation to their prior use of the neuropharmacological intervention, and optionally selected for further treatments appropriate to the patient response.

In disclosure which does not form an embodiment of the present invention there is provided a method of determining a patient's likelihood of developing one or more neurological disorders, comprising the steps of:
obtaining data indicative of electrical activity within the brain of the patient;
generating a network, based at least in part on the obtained data, said network comprising a plurality of nodes and directed connections between nodes, wherein the network is indicative of a flow of electrical activity within the brain of the patient;
calculating, for each node, a difference in a number and/or strength of connections into the node and a number and/or strength of connections out of the node; and
determining, using the calculated differences, the patient's likelihood of developing one or more neurological disorders.

The inventors have shown that even quite brief analysis using (for example) EEG of the brain can be used to potentially identify patients who are susceptible to one or more neurocognitive diseases (for example AD). Specifically, such individuals (patients or subjects, the terms are used interchangeably) may have a relatively high number of 'sinks', or sinks which are relatively strong, in the posterior lobes, and a relatively high number of 'sources', or sources which are relatively strong, in the temporal and/or frontal lobes. In apparently normal or prodromal subjects, in some examples, the method may be more sensitive than commonly used psychometric measures for determining such risk.

Optional features will now be set out. These are applicable singly or in any combination with any aspect of the disclosure.

The likelihood of a patient developing one or more neurological disorders may be referred to as a patient's susceptibility to one or more neurological disorders. The method may include a step of defining a state for each node, whereby a node is defined as either a sink or a source based on the calculated difference.

The network may be a renormalized partial directed coherence network. Any of the steps of the method may be performed offline, i.e. not live on a patient. For example, obtaining the data may be performed by receiving, over a network, data which has been previously recorded from a patient.

The data indicative of electrical activity within the brain may be electroencephalography data. The electroencephalography data may be β-band electroencephalography data. The data indicative of electrical activity within the brain may also be magnetoencephalography data or functional magnetic resonance imaging data.

Determining the patient's susceptibility may be performed using a machine learning classifier. For example, Markov models, support vector machines, random forest, or neural networks.

The method may include a step of producing a heat-map based, at least in part, on the states of the nodes, said heat-map indicating the location and/or intensity of nodes defined as sinks and nodes defined as sources within the brain of the patient. This representation of the defined nodes can aid (e.g. ergonomically) in the determination of the patient's susceptibility.

In determining the patient's susceptibility, a comparison may be made between the number and/or intensity of sources within the parietal and/or occipital lobes and the number and/or intensity of sinks within the frontal and/or temporal lobes. It has been seen experimentally that patients who are susceptible to one or more neurodegenerative diseases (and particularly Alzheimer's disease) have a relatively high intensity of sinks in the posterior lobes, and a relatively high intensity of sources in the temporal and/or frontal lobes.

The method may further comprise a step of deriving, using the states of the nodes, an indication of a degree of left-right asymmetry in the location and/or intensity of nodes in the brain corresponding to sinks and sources.

The neurological disorder may be a neurocognitive disease, which may be Alzheimer's disease.

The patient's susceptibility to one or more neurological disorders may be determined by comparing the number and/or intensity of nodes defined as sinks in the posterior lobe to a predetermined value, and/or comparing the number and/or intensity of nodes defined as sources in the temporal and/or frontal lobes to a predetermined value. A patient may be determined to be at a high risk of susceptibility if the number and/or intensity of nodes defined as sinks in the posterior lobe exceeds a predetermined value and/or if the number and/or intensity of nodes defined as sources in the temporal and/or frontal lobes exceeds a predetermined value (for example based on a 'control' subject or subjects established as having a low risk, or reference data (e.g. historical reference data) obtained from the same). Said another way, and more generally, a determination as to the susceptibility may be based on whether the patient has more and/or stronger sources and/or sinks in one region of the brain relative to another region of the brain. For example, if there are more and/or stronger sources in the temporal and/or frontal lobes than expected, and/or whether the patient has more and/or stronger sinks in the posterior lobe than expected based on data from control subjects, the patient may be determined as at risk of a neurodegenerative disorder. Such data from control subjects may have been established by longitudinal monitoring following base-line assessment.

The inventors have further observed that symptomatic treatment(s) increase activity outgoing from the frontal lobe compared to the non-medicated group.

The method according to this example may be used for assessing, testing, or classifying a subject's susceptibility to one or more neurological disorders for any purpose. For example, the score value or other output of the test may be used to classify the subject's mental state or disease state according to predefined criteria.

The subject may be any human subject. In one example, the subject may be one suspected of suffering a neurocognitive disease or disorder e.g. a neurodegenerative or vascular disease as described herein, or maybe one who is not identified as at risk.

In one example, the method is for the purpose of the early diagnosing or prognosing of a cognitive impairment, for example a neurocognitive disease, in the subject.

The disease may be mild to moderate Alzheimer's disease.

The disease may be mild cognitive impairment.

However the findings of the present inventors described herein have applicability to other neurocognitive diseases also. For example the disease may be a different dementia, for example vascular dementia.

The method may optionally be used to inform further diagnostic steps or interventions for the subject - for example based on other methods of imaging or invasive or non-invasive biomarker assessments, where such methods are known *per se* in the art.

In some examples, the method may be for the purpose of determining the risk of a neurocognitive disorder in the subject. Optionally, said risk may additionally be calculated using further factors, e.g. age, lifestyle factors, and other measured physical or mental criteria. Said risk may be a classification of "high" or "low" or may be presented as a scale or spectrum.

It will be apparent from the disclosure herein that in addition to assessing the likelihood of developing one or more neurological disorders, the same methodology can be used to assess the efficacy of a disease-modifying treatment to reduce said risk and/or treat said disease i.e. to assess the efficacy of a pharmaceutical for prophylaxis or treatment of the disease or disorder. This may optionally be in the context of a clinical trial as described herein, e.g. in comparison to a placebo, or other normal control.

Specifically, the disclosure herein indicates that the methods of the invention and the examples (e.g. based on EEG technology) can provide a powerful and sensitive measures of the disease impact on a subject. This opens up the opportunity to demonstrate the efficacy of a disease-modifying treatment in smaller groups of subjects (e.g. less than or equal to 200, 150, 100, or 50 in treatment and comparator arms) and over a shorter interval (e.g. less than or equal to 6, 5, 4, or 3 months) and in earlier stages of disease or less severe disease (e.g. prodromal AD, MCI or even pre-MCI) than is possible using currently available methods.

Thus, as discussed above, the method may be used with different patient cohorts in clinical trials of a neuropharmacological intervention e.g. a patient group (plurality of patients) may be a treatment group who have been diagnosed with the disease (for example early stage disease) treated with a putative disease modifying treatment vs. group treated with placebo.

Thus in one further example the method steps of the first example are used to determine disease status or severity in a patient, rather than determining a patient's likelihood of developing one or more neurological disorders. That status can in turn be monitored as part of clinical management or a clinical trial.

Thus in one further example which is not an embodiment of the invention there is provided a method of determining a patient response to a neuropharmacological intervention against a neurological disorder, comprising the steps, before the neuropharmacological intervention, of:
(a) obtaining data indicative of electrical activity within the brain of the patient;
(b) generating a network, based at least in part on the obtained data, said network comprising a plurality of nodes and directed connections between nodes, wherein the network is indicative of a flow of the electrical activity within the brain of the patient;
(c) calculating, for each node, a difference in a number and/or strength of connections into the node and a number and/or strength of connections out of the node; and
(d) determining, using the calculated differences, the patient's status in relation to the neurological disorder;
(e) repeating steps (a) - (d), after the neuropharmacological intervention, to determine a further status of the patient in relation to the neurological disorder; and
(f) determining, based on said first status and said second status (e.g. by comparing the two), the patient response to the neuropharmacological intervention.

Optionally steps (e) and (f) are repeated and subsequent statuses are used to determine the patient response over time.

Thus these methods of the first and further examples (and corresponding systems discussed hereinafter) can be used for both clinical trials and clinical management. In terms of clinical management, a high degree of certainty (for example, 70%, 80%, 90%, or 95% probability) that the electrical activity within the brain of the patient (e.g. as assessed using EEG) is abnormal in a "normal" person (i.e. presently undiagnosed) may be a strong indication for immediately starting dementia medication treatment. The EEG could also be used in at intervals, for example, 1, 2, 3, 4, 5, or 6 months to monitor response to treatment. Conversely, a person with lower probability of abnormal EEG (for example, 30%, 40%, 50%, 55%, or 60%) could be followed more closely at monthly, bimonthly, or trimonthly intervals. Further tests by other means appropriate to the disorder, such as are known in the art (e.g. assessment of biomarkers based on amyloid or tau PET or CSF) may optionally be used in conjunction with the method.

Optional features in relation to the methods of the first example apply mutatis mutandis to this example. ***

In a second aspect, the invention provides a system for determining patient response to a neuropharmacological intervention, the system comprising:
data acquisition means, configured to obtain structural neurological data from a plurality of patients before neuropharmacological intervention, said structural neurological data indicative of a physical structure of a plurality of cortical regions;
   wherein the physical structure is cortical thickness and/or surface area;
correlation matrix generation means, configured to generate a first correlation matrix from the structural neurological data by:
   assigning a plurality of structure nodes corresponding to cortical regions of the brain; and
   determining pair-wise correlations between pairs of the structure nodes based at least in part on corresponding data of the structural neurological data;
wherein the data acquisition means is also configured to obtain further structural neurological data from the plurality of patients after neuropharmacological intervention, said further structural neurological data being indicative of the physical structure of the plurality of cortical regions; and
the correlation matrix generation means is also configured to generate a second correlation matrix from the further structural neurological data by:
   determining pair-wise correlations between pairs of the structure nodes based at least in part on corresponding data of the further structural neurological data;
wherein the system further comprises either:
   display means, for presenting the first correlation matrix and second correlation matrix; or
   comparison means, for comparing the first correlation matrix and the second correlation matrix, and thereby determining patient response to the neuropharmacological intervention.

Optional features of the invention will now be set out. These are applicable singly or in any combination with any aspect of the invention.

By correlation matrix, it may be meant that a structural correlation network is generated which may then be represented by a matrix.

The values for the cortical thickness and/or surface area may be averaged values obtained from the structural neurological data. The structured neurological data may be acquired from magnetic resonance imaging (MRI) data or computed tomography data for each patient. The structural neurological data and the further structural neurological data are obtained at different points in time. As discussed herein, the structural neurological data may be obtained via magnetic resonance imaging, computed tomography, or positron emission tomography for each patient. These techniques are well known *per se* to those skilled in the art - see e.g. Mangrum, Wells, et al. Duke Review of MRI Principles: Case Review Series E-book. Elsevier Health Sciences, 2018, and "Standardized low-resolution electromagnetic tomography (sLORETA): technical details" Methods Find Exp. Clin. Pharmacol. 2002:24 Suppl. D:5-12; Pascual-Marqui RD etc.

The plurality of cortical regions may be at least 60, or at least 65. For example, 68. The cortical regions may, for example, be those provided by the Desikan-Killiany Atlas (Desikan et al. 2006).

The display means may provide each of the first correlation matrix and the second correlation matrix on a display, wherein correlation values in each correlation matrix are given a colour corresponding to the relative amplitude or strength of the correlation.

The verification means may be configured to determine a p-value for each pair-wise correlation, and compare the p-value for each pair-wise correlation, and may compare the p-value to a significance level, the correlation matrix generating means may be configured to use only p-values less than the corrected significance level when generating a correlation matrix. The significance level may be referred to as alpha ('α').

The comparison means may be configured to compare a number and/or density of inverse correlations in the first correlation matrix to a number and/or density of inverse correlations in the second correlation matrix. In making the comparison, groups of structure nodes corresponding to a same lobe may be identified, and the comparison made between the first and second correlation matrix may utilize the same lobe.

Assigning the plurality of structure nodes corresponding to cortical regions of the brain may further include defining groups which contain structure nodes corresponding to homologous or non-homologous lobes. The comparison means may be configured to compare the first correlation matrix and the second correlation matrix by comparing the number and/or density of correlations between different groups of structure nodes. Said another way, comparing the first and second correlation matrices may include comparing pairs of structure nodes which are non-homologous.

The comparison means may be configured to compare the first correlation matrix and the second correlation matrix by comparing the number and/or density of correlations between groups of structure nodes located respectively in the frontal lobe (anterior nodes) and the parietal and occipital lobes (posterior nodes). It has been found that, in examples of efficacious neuropharmacological intervention, the number and/or density of inverse correlations between anterior and posterior nodes decreases. As inverse correlations are postulated to indicate compensatory link formulation whereby atrophy in one node is associated with hypertrophy in a functionally linked node, it will be appreciated that a decrease in the number and/or density of inverse correlations indicates a decrease in the number of compensatory links.

In one embodiment, the patient response may be in the context of a clinical trial e.g. for assessing the efficacy of a pharmaceutical of a neurocognitive disease. Thus the patient group (plurality of patients) may be treatment group who have been diagnosed with the disease, or maybe a control ('normal') group. Ultimately the efficacy of the pharmaceutical may be assessed in whole or in part based on the patient group response determined in accordance with the present invention.

As explained in relation to the first aspect, the neurocognitive disease will generally be a neurodegenerative disorder causing dementia, for example a tauopathy.

The patients may have been diagnosed with the neurocognitive disease, for example Alzheimer's disease or behavioural-variant frontotemporal dementia. The disease may be mild or moderate Alzheimer's disease. The disease may be a mild cognitive impairment Diagnosis criteria and treatment of tauopathies, and these disorders, are discussed, for example, in WO2018/019823, and references cited therein.

The disease may be behavioural variant frontotemporal dementia (bvFTD). Diagnosis criteria and treatment of bvFTD is discussed, for example, in WO 2018/041739, and references cited therein.

As explained herein, the topology of the disturbance in structural network is different in the two disease conditions (AD and bvFTD) and both are different from normal aging. These changes appear to be adaptive in character, reflecting coordinated increases in cortical thickness and surface area that compensate for corresponding impairment in functionally linked nodes.

Thus, if a neuropharmacological intervention is efficacious, it is expected that the network organisation will be brought back towards a normal state. If the condition is treated at an early enough stage, the network organisation may be brought back to normal indicating arrest or reversal of the disease state. The system therefore provides an objective means of distinguishing disease-modifying treatments from symptomatic treatments as described above.

Typically, the neuropharmacological intervention will be a pharmaceutical intervention.

The neuropharmacological intervention may be a symptomatic treatment as described above..

For example, a disease modifying treatment may be an inhibitor of pathological protein aggregation such as a 3,7-diaminophenothiazine (DAPTZ) compound as described above.

A second example which is not an embodiment of the present invention there is discussed a system for determining a patient's susceptibility to one or more neurological disorders, the system comprising:
data acquisition means, configured to obtain data indicative of electrical activity within the brain of the patient;
network generating means, configured to generate a network based at least in part on the obtained data, said network comprising a plurality of nodes and directed connections between nodes, wherein the network is indicative of a flow the electrical activity within the brain of the patient;
difference calculation means, configured to calculate, for each node, a difference in a number and/or strength of connections into the node and a number and/or strength of connections out of the node; and either:
   display means, configured to display a representation of the calculated differences; or
   determination means, configured to determine using the calculated differences, the patient's susceptibility to one or neurological disorders.

As described above in relation to the first example, the inventors have shown that even quite brief analysis using (for example) EEG of the brain can be used to potentially identify patients who are susceptible to one or more neurocognitive diseases (for example AD).

The system can be used for both clinical trials and clinical management.

Thus in a further example there is provided a system as described above for determining a patient response to a neuropharmacological intervention against a neurological disorder, In this aspect the determination means system may be configured for determining, using the calculated differences, the patient's status in relation to the neurological disorder.

The system can be used to determine a further status of the patient after the neuropharmacological intervention, and optionally configured to determine, based on the first and one or more subsequent statuss, the patient response to the neuropharmacological intervention, as described above in relation to the corresponding method.

Other optional features of the second example will now be set out. These are applicable singly or in any combination with any second example.

The system may include state definition means configured to define a node as either a sink or a source based on the calculated difference.

The network may be a renormalized partial directed coherence network. The system may operate "offline", i.e. not live on a patient. For example, obtaining the data may be performed by receiving, over a network, data which has been previously recorded from a patient.

The data indicative of electrical activity within the brain may be electroencephalography data. The electroencephalography data may be β-band electroencephalography data. The data indicative of electrical activity within the brain may also be magnetoencephalography data or functional magnetic resonance imaging data.

The determination means may be configured to use a machine learning classifier to determine the patient's susceptibility to one or more neurological disorders. For example, Markov models, support vector machines, random forest, or neural networks.

The display means may be configured to present a heat map indicative of the location and/or intensity of nodes defined as sinks and nodes defined as sources within the brain. This representation of the defined nodes can aid (e.g. ergonomically) in the determination of the patient's susceptibility.

The system may further comprise a heat map generating means, configured to produce a heat map based at least in part on the states of the nodes, said heat-map indicating the location and/or intensity of nodes defined as sinks and nodes defined as sources within the brain of the patient. This representation of the defined nodes can aid (e.g. ergonomically) in the determination of the patient's susceptibility.

The determination means may compare the number/and or intensity of sources within the parietal and/or occipital lobes as compared to the number and/or intensity of sinks within the frontal and/or temporal lobes. It has been seen experimentally that patients who are susceptible to one or more neurodegenerative diseases (and particularly Alzheimer's disease) have a relatively high number and/or intensity of sinks in the posterior lobes, and a relatively high number and/or intensity of sources in the temporal and/or frontal lobes.

The system may further comprise an asymmetry map generation means, configured to derive, using the states of the nodes, an indication of a degree of left-right asymmetry in the location and/or density of nodes in the brain corresponding to sinks and sources.

The neurological disorder may be a neurocognitive disease, which is optionally Alzheimer's disease.

The determination means may compare the number and/or intensity of nodes defined as sinks in the posterior lobe to a predetermined value, and/or compare the number and/or intensity of nodes defined as sources in the temporal and/or frontal lobes to a predetermined value. The determination means may determine a patient to be at a high risk of susceptibility if the number and/or intensity of nodes defined as sinks in the posterior lobe exceeds a predetermined value and/or if the number and/or intensity of nodes defined as sources in the temporal and/or frontal lobes exceeds a predetermined value. Said another way, and more generally, a determination as to the susceptibility may be based on whether the patient has more and/or stronger sources and/or sinks in one region of the brain relative to another region of the brain. For example, if there are more/and/or stronger sources in the temporal and/or frontal lobes than expected, and/or whether the patient has more and/or stronger sinks in the posterior lobe than expected, then the patient may be determined as at risk of a neurodegenerative disorder.

The inventors have further observed that symptomatic treatment(s) increase activity outgoing from the frontal lobe compared to the non-medicated group.

The system of the invention according to this example may be used for assessing, testing, or classifying a subject's susceptibility to one or more neurological disorders for any purpose. For example, the score value or other output of the test may be used to classify the subject's mental state or disease state according to predefined criteria.

The subject may be any human subject. In one example, the subject may be one suspected of suffering a neurocognitive disease or disorder e.g. a neurodegenerative or vascular disease as described herein, or maybe one who is not identified as at risk.

In one example, the system is for the early diagnosing or prognosing of a cognitive impairment, for example a neurocognitive disease, in the subject as described above.

The system may optionally be used to inform further diagnostic steps or interventions for the subject - for example based on other systems for imaging or invasive or non-invasive biomarker assessments, where such systems are known *per se* in the art.

In some examples, the system may be for determining the risk of a neurocognitive disorder in the subject. Optionally, said risk may additional be calculated using further factors, e.g. age, lifestyle factors, and other measured physical or mental criteria. Said risk may be a classification of "high" or "low" or may be presented as a scale or spectrum.

As with the methods described herein, the system may be used in the context of a clinical trial, to assess the efficacy of a neuropharmacological intervention. The system may be used to demonstrate the efficacy of a disease-modifying treatment, for example LMTM, in a relatively small number of subjects (e.g. 50) over a relatively short time scale (e.g. 6 months) and in early disease stages (for example mild cognitive impairment or possible pre-mild cognitive impairment).

Further aspects of the present invention provide: a computer program comprising executable code which, when run on a computer, causes the computer to perform the method of the first or second aspect; a computer readable medium storing a computer program comprising code which, when run on a computer, causes the computer to perform the method of the first or second aspect; and a computer system programmed to perform the method of the first or second aspect. For example, a computer system can be provided, the system including: one or more processors configured to: perform the method of the first or second aspect. The system thus corresponds to the method of the first or second aspect. The system may further include: a computer-readable medium or media operatively connected to the processors, the medium or media storing computer executable instructions corresponding to the method of the first or second aspects.

### Brief Description of the Drawings

Embodiments of the invention and examples useful for understanding the invention will now be described by way of example with reference to the accompanying drawings in which:
Figure 1 shows an example of the Desikan-Killiany brain Atlas;
Figure 2 shows an example cortical surface area correlation matrix with pairwise correlations grouped by lobe for a group of subjects diagnosed with behavioral variant frontotemporal dementia;
Figures 3A - 3C show, respectively group-based cortical thickness correlation networks depicted as pair-wise correlation matrices for: (i) a group of HE subjects, (ii) a group of bvFTD subjects, and (iii) a group of AD subjects;
Figures 4A - 4C show, respectively group-based surface area correlation networks depicted as pair-wise correlation matrices for (i) a group of HE subjects, (ii) a group of bvFTD subjects, and (iii) a group of AD subjects;
Figure 5 shows plots of mean edge strength of the cortical thickness correlation network averaged over brain lobes and compared between HE, bvFTD, and AD groups, the upper plot for networks of positive correlations and the lower plot for networks of inverse correlations;
Figure 6 shows plots of mean edge strength of the surface area correlation network averaged over brain lobes and compared between HE, bvFTD, and AD groups, the left plot for networks of inverse correlations and the right plot for positive correlations;
Figure 7 shows plots of node degrees of the cortical thickness correlation network averaged over brain lobes across HE, bvFTD, and AD groups, the upper plot for networks of positive correlations and the lower plot for networks of inverse correlations;
Figure 8 shows a plot of node between-lobes participation indexes of the cortical thickness correlation network averaged over brain lobes and compared across HE, bvFTD, and AD groups for positive correlations;
Figure 9 shows plots of node degrees of the surface area correlation network averaged over brain lobes and compared across HE, bvFTD, and AD groups, the upper plot for networks of positive correlations and the lower plot for networks of inverse correlations;
Figure 10 shows plots of node between-lobes participation indexes of the surface area correlation network averaged over brain lobes and compared across HE, bvFTD, and AD groups, the upper plot for networks of positive correlations and the lower plot for networks of inverse correlations;
Figure 11 shows a visualization in brain space of hubs in the cortical thickness network for, in the upper plot, positively correlated nodes and, in the lower plot, inversely correlated nodes for the HE, bvFTD, and AD groups;
Figure 12 shows a visualization in brain space of hubs in the surface area network for, in the upper plot, positively correlated nodes and, in the lower plot, inversely correlated nodes for the HE, bvFTD, and AD groups;
Figure 13 shows a visualization in brain space of the interaction between cortical thickness and cortical surface area positive networks for the HE, bvFTD, and HE groups;
Figure 14 shows histograms of the retained edges in the cortical thickness (upper three plots) and surface area (lower three plots) correlation networks;
Figure 15 is a plot showing the distribution of the modularity index (Q) in regional cortical thickness correlation networks generated on 100 surrogate data sets;
Figure 16 shows binarised correlation matrices of the cortical thickness network (upper three figures) and surface area (lower three figures) for the HE, bvFTD, and AD groups, white representing significant positive correlations and black representing significant inverse correlations;
Figure 17A - 17D show correlation matrices at baseline (i.e. week 01) according to treatment status with symptomatic drugs for AD (cholinesterase inhibitors and/or memantine) ach0 indicting no treatment and ach1 indicating the presence of such treatment;
Figures 18A - 18D show plots of node degrees of non-homologous inter-lobar correlations at baseline node degrees according to treatment status with symptomatic AD drugs (acetylcholine esterase inhibitors and/or memantine);
Figures 19A and 19B show temporally separated cortical thickness correlation matrices at baseline (week 01) and after 65 weeks (week65) in patients receiving 8 mg/day LMTM in combination with symptomatic treatments;
Figures 20A - 20D show plots of positive and inverse non-homologous inter-lobar node degree of correlations in cortical thickness (CT) and surface area (SA) at baseline and after 65 weeks in patients receiving 8 mg/day LMTM in combination with symptomatic treatments;
Figures 21A and 21B show temporally separated cortical thickness correlation matrices at base line (week01) and after 65 weeks (week65) in patients receiving 8 mg/day LMTM as monotherapy (i.e. not in combination with symptomatic AD treatments);
Figures 22A and 22B show plots of non-homologous inter-lobar node degree for the cortical thickness correlation network at baseline and after 65 weeks in patients receiving 8 mg/day LMTM in combination as monotherapy (i.e. not in combination with symptomatic AD treatments);
Figures 23A and 23B show temporally separated surface area correlation matrices at baseline (week01) and after 65 weeks (week65) in patients receiving 8 mg/day LMTM as monotherapy (i.e. not in combination with symptomatic AD treatments);
Figures 24A and 24B show plots of non-homologous inter-lobar node degree for the surface area correlation network at baseline and after 65 weeks in patients receiving 8 mg/day LMTM in combination as monotherapy (i.e. not in combination with symptomatic AD treatments);
Figures 25A - 25D show temporally separate cortical thickness correlation matrices for both AD (clinical dementia rating 0.5, 1, and 2) at baseline and after 65 weeks of treatment with LMTM 8mg/day as monotherapy and the elderly control group (HE) to show normalization of the matrix following treatment;
Figures 26A - 26D show temporally separate surface area correlation matrices for both AD (clinical dementia rating 0.5, 1, and 2) at baseline and after 65 weeks of treatment with LMTM 8mg/day as monotherapy and the elderly control group (HE) to show normalization of the matrix following treatment;
Figures 27A - 27D show temporally separate cortical thickness correlation matrices for both AD (clinical dementia rating 0.5) at baseline and after 65 weeks of treatment with LMTM 8 mg/day as monotherapy and the elderly control group (HE) to show normalization of the matrix following treatment;
Figures 28A - 28D show temporally separate surface area correlation matrices for both AD (clinical dementia rating 0.5) at baseline and after 65 weeks of treatment with LMTM 8 mg/day as monotherapy and the elderly control group (HE) to show normalization of the matrix following treatment;
Figure 29 shows an example of resting electroencephalography data;
Figure 30 shows an example of a directed network derived from electroencephalography data;
Figure 31 shows, schematically, a determination of node state as the difference between inbound flow of electrical activity and outbound flow of electrical activity;
Figure 32 shows a heat map of the location of net sinks (yellow / red) and net sources (blue) within the brain of a group of subjects;
Figure 33 shows a heat map indicating the asymmetry in the distribution of sources and sinks between the left and right sides of the heat map in Figure 32;
Figure 34 shows a heat map of the location of sources and sinks within the brain of a group of subjects diagnosed with Alzheimer's disease;
Figure 35 shows a heat map of the location of sources and sinks within the brain of a group of subjects who have not been diagnosed with Alzheimer's disease (i.e. paired volunteers);
Figure 36 shows a heat map of the location of sources and sinks within the brain of a subject who has not been diagnosed with Alzheimer's disease (i.e. a paired volunteer);
Figure 37 shows a heat map of the location of sources and sinks within the brain of a subject diagnosed with Alzheimer's disease;
Figure 38 shows a heat map of the location of sources and sinks within the brain of a group of subjects who are determined to be at risk of dementia or cognitive decline, for example due to having Alzheimer's disease;
Figure 39 shows a heat map of the location of sources and sinks within the brain of a group of subjects who are determined to not be at risk of Alzheimer's disease;
Figure 40 is a box and whisker plot comparing sources and sinks in EEG networks from frontal and posterior brain regions at group level in subjects at risk of AD and not at risk of AD;

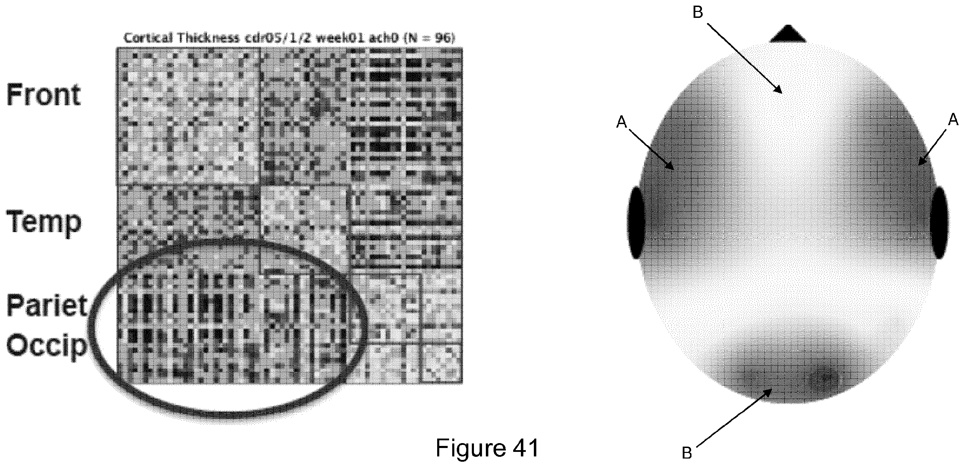

Figure 41 shows a comparison between a cortical thickness correlation matrix for the AD group showing increase in strength and number of significant inverse non-homologous between-lobe correlations (left) and a heat map of the location of sources and sinks within the brain of a group of subjects who have been diagnosed with AD showing correspondence between increase in compensatory structural inverse non-homologous correlations in cortical thickness directed to posterior brain regions and increase in strength and number of inbound connections to the posterior regions of the brain as a sink shown by rPDC coherence analysis of resting state EEG;
Figure 42 shows a comparison between a surface area correlation matrix for the HE group and a heat map of the location of sources and sinks within the brain of a group of healthy elderly subject showing correspondence between relative lack of compensatory structural inverse non-homologous correlations in cortical thickness directed to posterior brain regions and reduction in number and strength of inbound connections to the posterior regions of the brain as a sink shown by rPDC coherence analysis of resting state EEG;
Figure 43 is a box and whisker plot showing quantitative differentiation of mild AD from elderly controls;
Figure 44 shows three heat maps comparing medicated and non-medicated AD patients and paired volunteers at group level; and
Figure 45 is a group level network box and whisker plot comparing medicated and non-medicated AD patients, and paired volunteers.

### Detailed Description and Further Optional Features

Aspects and embodiments of the present invention will now be discussed with reference to the accompanying figures. Further aspects and embodiments will be apparent to those skilled in the art.

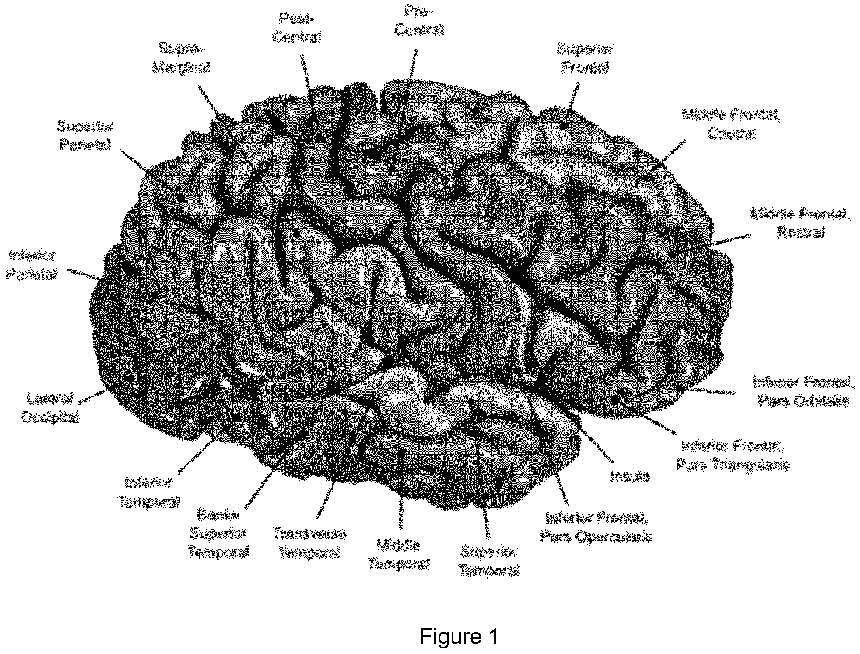

Figure 1 gives an example of the Desikan-Killiany brain Atlas. The Desikan-Killiany brain Atlas divides the human cerebral cortex on MRI scans into gyral based regions of interest.

Whilst 18 regions are shown in the figure, the full Desikan-Killiany brain Atlas divides the human cortex into 68 regions of interest.

The subjects discussed in this document participated in three global Phase 3 clinical trials which have now been completed. Two of the clinical trials were in mild to moderate AD (Gauthier et al., 2016; Wilcock et al., 2018) and the third was from a large study of bvFTD (Feldman et al., 2016). Comparable data was available from well characterised healthy elderly (HE) subjects participating in an ongoing longitudinal study of the Aberdeen 1936 Birth Cohort (ABC36) (Murray et al., 2011). In all, in the examples discussed herein, there were 628 subjects with 213 in each of the dementia groups and 202 healthy elderly subjects. The bvFTD patients were diagnosed according to the International Consensus Criteria for bvFTD, with mild severity on the Mini-Mental State Examination (MMSE) score of 20 - 30 inclusive. AD patients were diagnosed according to the criteria from the National Institute of Aging and the Alzheimer's Association, with mild to moderate severity defined by an MMSE score of 14 - 26 (inclusive) and a Clinical Dementia Rating (CDR) total score of 1 or 2. They were drawn from the corresponding larger group (N = 1132) to match the number of participants in the bvFTD group. The healthy elderly (HE) subjects were selected from a well characterised Aberdeen 1936 Birth Cohort.

The multi-side source imaging data sets, used to generate the correlation matrices discussed below, were standard T1-weighted MRI images acquired using equivalent manufacturer specific 3DT1 sequences. The data from trial patients were pooled to permit overall group-wise comparisons. The train scanners were limited to 1.5T and 3T (30%) field strengths from three manufacturers (Philips, GE, and Siemens). MRI images in the ABC36 cohort were all acquired using the same (Philips) 3T scanner. The images were processed using an automated process pipeline implemented in a manner known *per se.* In addition to the volume-based methods of image processing, the pipeline produces surface-based regional measurements of cortical morphology such as thickness, the local curvature or surface area. An example of an automated processing pipelines suitable for the above methods is FreeSurfer v5.3.0 available from the Athinoula A. Martinos Centre for Biomedical Imaging at Massachusetts General Hospital.

Surface area was calculated from the imaging data sets using triangular tessellation of the grey/white matter interface and white matter/cerebrospinal fluid boundary (referred to as the pial surface). The cortical thickness was calculated as an average of the distance from the white matter surface of the closest point on the pial surface, and from that point back to the closest point to the white matter surface. A parcellation scheme, known *per se,* was used to extract cortical thickness and surface area of 68 cortical regions from both hemispheres based on the Desikan-Killiany Atlas. A list of regions and their lobar assignment is given in Table A.1 in Annex A.

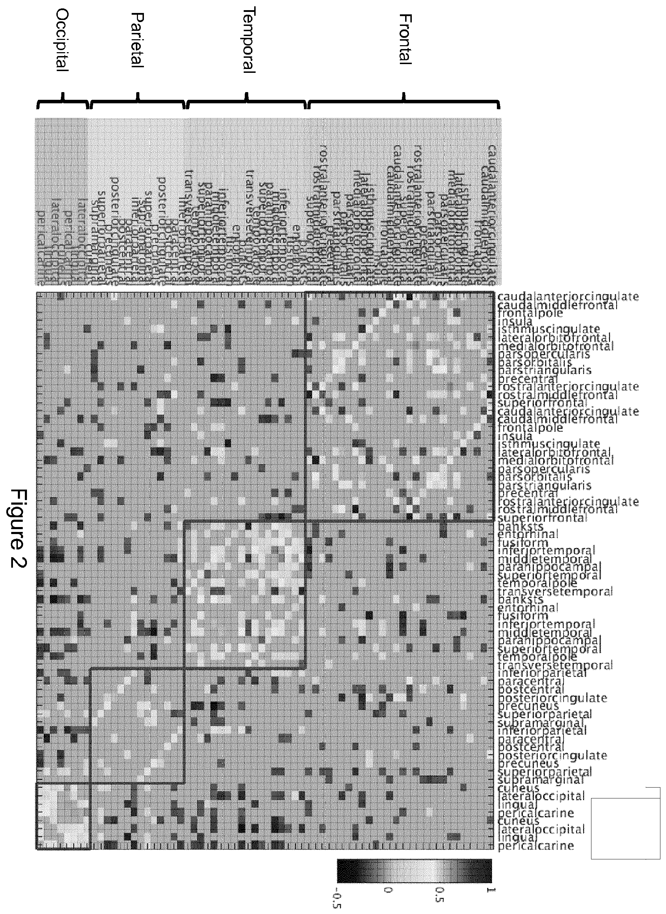

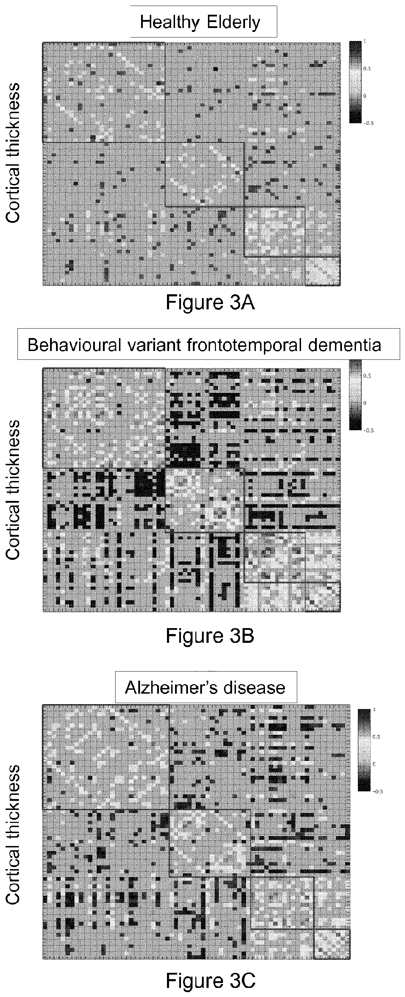

Figure 2 shows a cortical surface area correlation matrix for a group of subjects diagnosed with bvFTD. Each matrix element represents correlation strength ('edge strength') between 68 pairs of cortical surface areas from the Desikan-Killiany Atlas. The intensity bar to the right indicates correlation / edge strength. Sixty eight cortical surface regions (network nodes) are ordered according to their affiliations with the frontal, temporal, parietal, and occipital lobes. Single lobe regions are enclosed within squares and ordered from top to bottom / left to right: frontal, temporal, parietal, and occipital. In essence, the correlation matrix represents a network constructed from the partial correlations between 68 pairs of cortical thicknesses. Figures 3A - 3C show cortical surface area correlation matrices for, respectively, healthy elderly, behavioural variant frontotemporal dementia subjects, and Alzheimer's disease subjects. Marked differences can be seen between the healthy elderly and both the bvFTD and AD subjects. Notably, within lobe correlations increased in strength significantly for bvFTD and AD subjects. Further, the number of inverse correlations increased between non-homologous nodes. As can be seen, HE subjects have sparse correlations, and these are mostly positive correlations between homologous lobes. Whereas, both bvFTD and AD have significantly increased numbers of nodes linked by positive and inverse correlations compared with the HE group. Increase in the number of correlations in both forms of dementia can be between the same lobes (homologous, mainly positive) or between different lobes (non-homologous, mainly negative). Generally, inverse between-lobe non-homologous correlations are highly abnormal. Also, it can be seen that bvFTD particularly is associated with a higher density of inverse non-homologous correlations in cortical thickness.

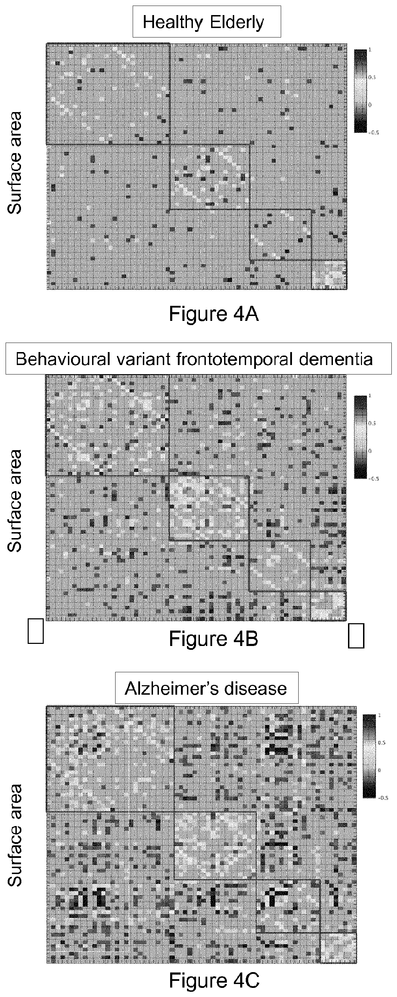

Figures 4A - 4C show surface area correlation matrices for, respectively, healthy elderly, behavioural variant frontotemporal dementia subjects, and Alzheimer's disease subjects. Noticeable differences can be seen between the healthy elderly and both bvFTD and AD subjects. Further, it should be noted that AD is particularly associated with a higher density of inverse non-homologous correlations in surface area.

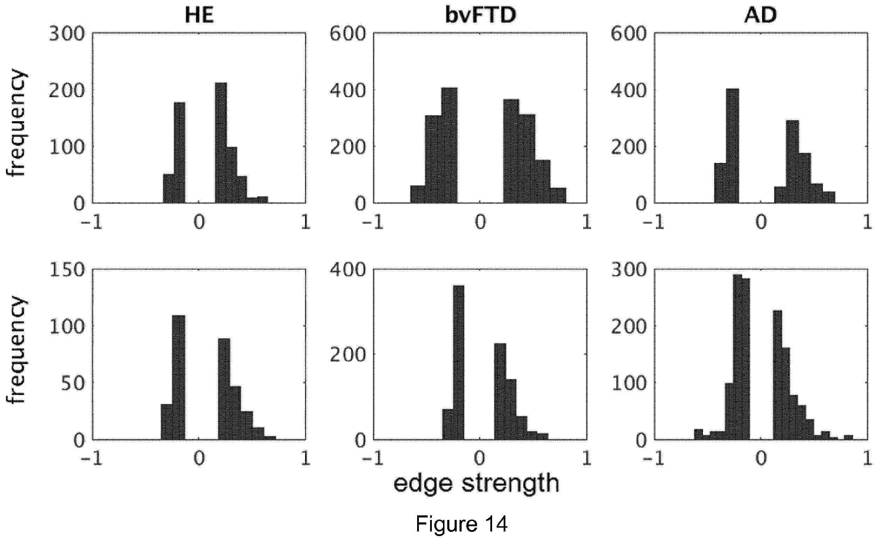

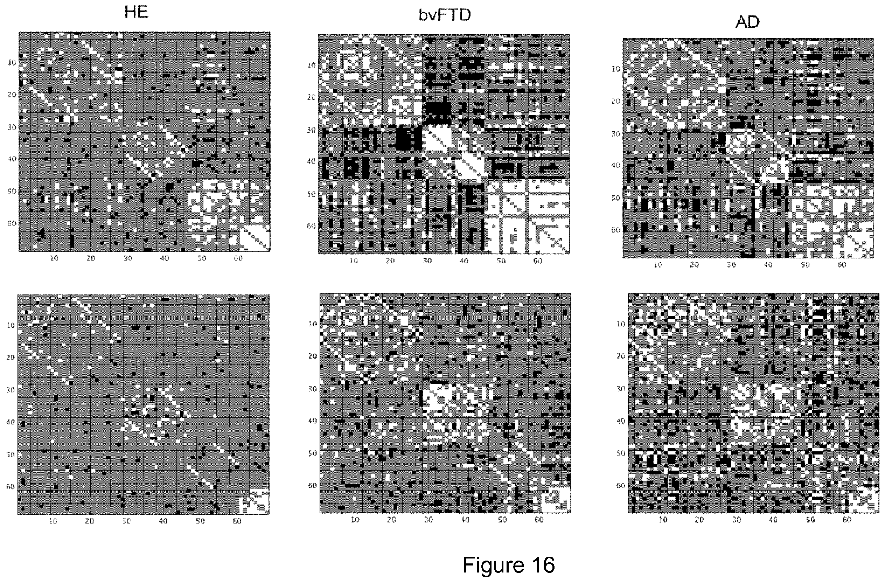

In these figures, zero entries correspond to non-significant correlations. It was found that the significant network correlations had both positive and negative values (see Figures 14 and 16 for the illustration). Because of the number of significant inverse correlations and the apparent increase in correlation strength of the networks in bvFTD and AD relative to the HE group, sub-networks of significant positive and inverse correlations were considered separately. Given the apparent differences in lobar network structure according to diagnostic group, an attempt was made to determine whether these differences could be quantified.

The networks, represented in these figures as correlation matrices, can be constructed by correlating either surface area or cortical thickness across all subjects within a particular diagnostic category (i.e. HE, bvFTD, and AD). A cortical region (as defined by the Desikan-Killiany brain Atlas) represents a node and a pair-wise correlation between nodes represents a graph edge or link/connection was constructed correlating either SA or CT across all participants within each diagnostic category. Each correlation matrix was calculated based on S x N array containing N regional CT/SA values from S subjects within each group. In this way, six N x N (e.g. 68 x 68) correlation matrices were obtained (one CT or SA structural correlation matrix for each study group). The matrix element *eᵢⱼ* is the value of the partial correlation between the region *i* and *j* (*i, j* = 1, 2, ... N) (i.e. between vectors *xᵢ* and *xⱼ* that contain regional measurements from subjects within each group). The partial correlations were calculated as linear, Pearson's correlation coefficients between pairs of *xᵢ* and *xⱼ* after first removing the effects of all other regions m ≠ (*i;j*) and then adjusting both *xᵢ* and *xⱼ* for controlling variables (stored in a separate array S x C, where C represents the number of controlling variables). This means that prior to correlation analysis a linear regression was performed on every *xᵢ* to remove the effect of age, gender, and mean CT (mean cortical thickness of all areas) or total surface area (sum of overall surface areas). Self-correlations (represented by the main matrix diagonnetwork measures were calculated on the lower triangular part of the matrix. The partial correlation, *eᵢⱼ* (i.e. edge weight), can be calculated according to the following general equations: ${e}_{ij} = {ρ}_{i} ≡ corr \left({x}_{i},{x}_{j}\left|{x}_{c}\right.\right)$

Where *x_{i;j}* denotes an array of variables and *x_{c}* denotes any subset of conditioning variables. To arrive at this general form of the partial correlation, the process begins from *i, j, c* = 1, 2, 3: ${ρ}_{12 ⋅ 3} = \frac{{ρ}_{12} - {ρ}_{13} {ρ}_{23}}{\left(1-{ρ}_{13}^{2}\right) {\left(1-{ρ}_{23}^{2}\right)}^{\frac{1}{2}}}$

Hence, for any subset of c of conditioning variables: ${ρ}_{12 ⋅ 3 c} = \frac{{ρ}_{12 ⋅ c} - {ρ}_{13 ⋅ c} {ρ}_{23 ⋅ c}}{\left(1-{ρ}_{13 ⋅ c}^{2}\right) {\left(1-{ρ}_{23 ⋅ c}^{2}\right)}^{1 / 2}}$

In some examples, to verify that the network retained only statistically significant correlations, the calculated correlation coefficients were adjusted for multiple tests using the False Discovery Rate (FDR) procedure as set out in Storey, 2002. The FDR procedure tests each calculated p-value (from the pair-wise correlation calculation) against a corrected significance level, in this example α = 0.05, and accepts only p-values smaller than the adjusted significance level as truly significant. Those pair-wise correlations that did not pass the FDR test may be set to zero; otherwise, all non-zero correlations, whether positive or negative, were retained (see Figure 14, discussed in more detail below).

In this way, a 68 × 68 correlation matrix can be constructed for either CT or SA in each clinical group, which represents the structural correlation network for either surface area of cortical thickness. A matrix element quantities the strength of the correlation between cortical regions for either cortical thickness or surface area and it does not in itself represent an actual physical connection. In the context of structural correlation network analysis in neurodegenerative disorders, such correlations are considered to imply either a co-atrophy relationship (if positive) or an inverse atrophy/hypertrophy relationship (if negative) between brain regions.

With regards to the structural correlation networks and/or matrices generated using the above methods, it is useful to use the following measures to compare the structural network properties of the three clinical groups: edge strength, node degree, node within-module degree z-score, and participation index. Edge strength and node degree represent two basic networks attributes; they respectively quantify the correlation strength between nodes and the number of pairwise correlations for each node. To assess whether cortical lobes represent modules, two network measures were utilised which assess modularity in network interactions, namely within-module degree z-score and participation index. All measures (except node degree) were computed on weighted graphs and where estimated as averages across the four lobes (described below). The measures were computed on either binary or weighted graphs (as discussed below). From purely theoretical studies, it is known that the calculated topological properties of a network depend on the choice of the threshold value (van Wijk et al. 2010). In this document, a fixed threshold for each group-based correlation matrix is chosen.

### Node Degree

Node degree, *kᵢ*, represents the number of significant correlations for each node in the network. In general, node degree is calculated from a binarised correlation matrix where each significant correlation in the matrix is replaced with either 1 if it is significant or with 0 if it is not. Examples of binarised matrices are shown in Figure 16. The binarised matrices can also be referred to as adjacency matrices. In Figure 16 the upper three plots correspond to cortical thickness and the lower three plots corresponding respond to surface area. Significant positive correlations are shown in white, whereas significant inverse correlations are shown in black.

The degree of a node *i*, i.e. the number of significant links connected to a node, can be calculated as: ${k}_{i} = {\sum }_{j = 1}^{N} {a}_{ij}$ Eqn. 1 where *N* is the number of nodes, and *aᵢⱼ* represents the connection between nodes *i* and *j* having a value of 1 if there is a direct connection between nodes and 0 otherwise.

### Modularity Index

Node participation index and within-module degree z-score assess the role of a node according to modules. Network modules (also known as community structures) represent densely connected sub-graphs of a network, i.e. subsets of nodes within which network connections are denser, and between which connections are sparser. It is useful to examine the modular organisation of frontal, temporal, parietal, and occipital divisions of cortical thickness or surface area network as defined as modules. Since these lobar divisions of the cortical surface area are not necessarily modular in themselves, it may be necessary to first test whether lobar divisions are intrinsically modular. In one example, this may be done by calculating the modularity index (Q) of the networks according to each lobe. The modularity index quantifies the observed fraction of within- module degree values relative to those expected if connections were randomly distributed across the network. Since the constructed cortical thickness and surface area networks contain both positive and negative edge strengths, it is possible to use an asymmetric generalization of the modularity quality function. For example, as introduced in Rubinov and Sporns (2011): $Q = \frac{1}{{v}^{+}} {\sum }_{ij}^{N} \left({ω}_{ij}^{+}-{e}_{ij}^{+}\right) {δ}_{{M}_{i} , {M}_{j}} - \frac{1}{{v}^{+} + {v}^{-}} {\sum }_{ij}^{N} \left({ω}_{ij}^{-}-{e}_{ij}^{-}\right) {δ}_{{M}_{i} , {M}_{j}}$ Eqn. 2

Where ${ω}_{ij}^{+}$ is equal to the *i, j*-th element of the correlation matrix, i.e. the strength of the pair-wise correlation between cortical regions, *ωᵢⱼ* if *ωᵢⱼ* > 0 and is equal to zero otherwise.

Similarly, *ω_{i̅j}* is equal to -*ωᵢⱼ* if *ωᵢⱼ* > 0 and is equal to zero otherwise. The term ${e}_{ij}^{\pm } = {s}_{i}^{\pm } {s}_{j}^{\pm } / {v}^{\pm }$ stands for the expected density of positive or negative connection weights given a strength-preserved random null model, where ${s}_{i}^{\pm } = {\sum }_{i}^{N} {ω}_{ij}^{\pm }$ and ${v}^{\pm } = {\sum }_{ij}^{N} {ω}_{ij}^{\pm }$. The Kronecker delta function *δ_{Mᵢ,Mⱼ}* is equal to one when the *i, j*-th nodes are within the same module and is equal to zero otherwise. The performance of a given separation of networks into modules was tested by applying a community detection function known *per se* in the art, while employing the vector of the node's affiliation with the particular node as the initial community affiliation vector.

It was found that lobar organisation of the cortical surface into frontal, parietal, temporal, and occipital divisions is in fact modular (see Annex A). Accordingly, it is then possible to calculate the contribution of individual nodes to lobar modules as the node participation index and the within-modules z-score, which is referred to as node between-lobes participation index and node within-lobe z-score.

### Node between-lobes participation index

In general, the participation index *p* assesses inter-modular connectivity. It may be considered the ratio of within-lobe node edges to all other lobar modules in the network, where node *pᵢ* tends to 0 if the node has links exclusively within its own module, and tends to 1 if the node links exclusively outside of its own module. The weighted network participation is calculated by: ${p}_{i}^{w} = 1 - {\sum }_{m ∈ M} {\left(\frac{{k}_{i}^{w} \left(m\right)}{{k}_{i}^{w}}\right)}^{2}$ Eqn. 3 **where** *M* **is** the set of modules and ${k}_{i}^{w} \left(m\right)$ is the weighted number of links of the i-th node to all other nodes in module m - inter-modular degree and ${k}_{i}^{w}$ is the total degree of the i-th node. Within this document, the term between-lobes participation is used for this network measure.

### Node within-lobe degree z-score

The complement of the between lobes participation index is the normalised within-lobe degree, *zᵢ*, which assesses intra-lobar connectivity by means of z-score i.e. by the normalised deviation of the inter-lobar degree of a node with the respective mean degree distribution. Therefore, node within-lobe z-score, *zᵢ*, is large for a node with more intra-modular connections relative to the inter-modular mean connectivity. For networks in which correlation strengths are preserved, the node within-module degree z-score is calculated as: ${z}_{i}^{w} = \frac{{k}_{i}^{w} \left({m}_{i}\right) - {k}^{- w} \left({m}_{i}\right)}{{σ}^{{k}^{w}} \left({m}_{i}\right)}$ Eqn. 4 where ${k}_{i}^{w} \left(m\right)$ is as above, ${k}_{i}^{- w} \left({m}_{i}\right)$ is the mean of the within module *mᵢ* degree distribution, and *σ^{k_{w}}(mᵢ*) is the standard deviation of the within module *mᵢ* degree distribution.

### Node role in network modular organisation

Node role with the modular lobar organisation depends on its position in the *z*-*pᵢ* parameter space. There are four possible roles that anode can have in the network, which are assigned on the basis of higher than average measures of nodal properties. It is useful to consider two of these roles, so called connectors or global network hubs (that have high between-lobes participation and high within-lobe degree z-score) and so called provincial hubs (that have high within-lobe degree z-score and low between-lobes participation). The thresholds for high and low values of *zᵢ* and *pᵢ* were set above 1.5 and 0.05, respectively.

### Statistical analysis

Statistical differences in demographic and cognitive scores in subjects were assessed using either one-way analysis of variance or two-tailed t-tests. The data was checked for normality of distribution using a one-sample Kolmogorov-Smirnov test. A chi-square test was used to test for differences in distribution of males and females between the groups. Statistical differences in global network correlation strength according to diagnostic groups were tested using one-way analysis of variance for unbalanced sample size (to account for an uneven number of significant correlations across the networks). The node degree, within-lobe z-score, *zᵢ* , and between-lobes participation index, *pᵢ*, were compared across the groups using the Kruskal-Wallis test, a non-parametric one-way analysis of variance test. Results were reported as significant at the level *p* < 0.05.

### Results

Table 1, below, shows demographic, cognitive, and mean CT and SA for each group according to clinical diagnosis. The 3 groups different significantly by age, AD patients being older than HE and bvFTD (p < 10⁻⁴ in all tests). Significant differences were also seen in cognitive scores on the MMSE scale, AD patients being the most impaired and bvFTD more impaired than HE subjects (p < 10⁻⁴ in all tests). The mean CT and total SA differed across groups.

**Table 1**

| | Gender M/F | Age (years) mean(sd) | MMSE mean(sd) | CT (mm) mean(sd) | Total SA (×10⁵mm²) mean(sd) |
|---|---|---|---|---|---|
| HE | 106/95 | 64(1)^{b} | 29(1)^{a,b} | 2.43(0.1)^{a,b} | 106.9(1.4)^{a,b} |
| bvFTD | 136/77 | 64(8)^{c} | 24(4)^{c} | 2.20(0.15) | 102.8(1.5) |
| AD | 105/108 | 71(10) | 21(4)^{c} | 2.18(0.15) | 102.5(1.6) |

Abbreviations: HE - healthy elderly, bvFTD - behavioural variant frontotemporal dementia, AD - Alzheimer's disease, M - males, F - females, MMSE - Mini-mental-state examination, CT - Cortical thickness, SA - Surface area. Significant differences between groups: a - HE/bvFTD, b - HE/AD, c - bvFTD/AD (p < 0.05).

The differences between HE and both patient groups were significant in terms of both mean CT (p < 10⁻⁴, in both tests) and total SA (p < 0.003 in both tests), but bvFTD and AD groups did not differ from each other. The mean CT and total SA values averaged by brain lobes are given in Table A.3 in Annex A. Therefore, although AD and bvFTD differ in terms of lobar distribution of pathology, age and severity of cognitive impairment, neither overall extent of cortical thinning or change in mean surface area provide a means of distinguishing between the two conditions.

### Lobar properties of structural correlation network

As the definition of correlation-based network organisation depends on the choice of the threshold value, it is useful to ensure that the networks defined herein were non-random in their global topology by calculating the density/sparsity value (*κ*). Brain networks are considered to show non-random (small-world) topology if *κ* > 0.1, which was the case for all networks considered here. It is also useful to ensure that inverse correlations were not omitted after thresholding (see Figure 14). Therefore, all positive and inverse CT and SA correlation networks considered herein are non-random. See also Table A.3 for global values for *κ* for CT and SA in the three groups.

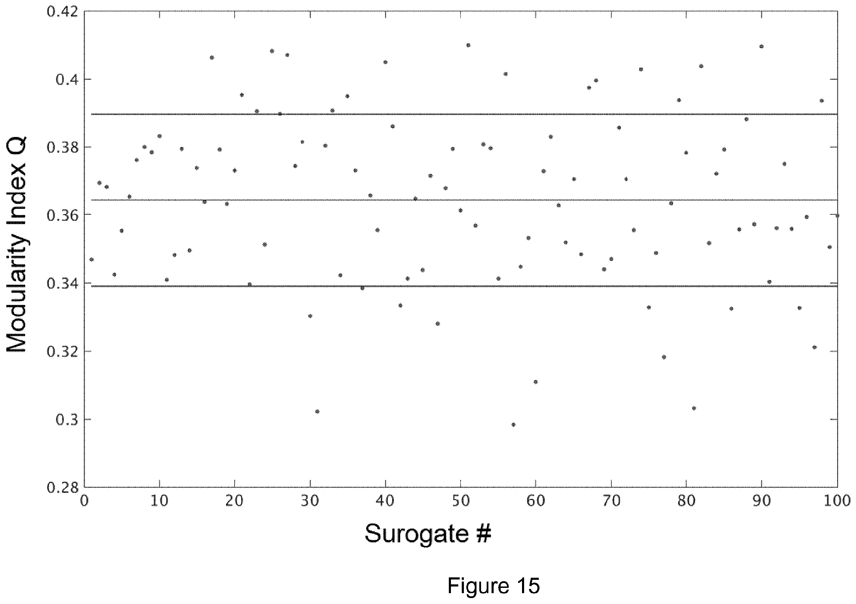

Using the modularity index, an investigation was performed to determine whether cortical lobes as conventionally defined correspond to network modules in the CT network. It was found that only two homologue pairs (posterior cingulate and precentral cortex) in the CT network and two homologue pairs in the SA network (posterior cingulate cortex and paracentral and right banks of the superior temporal sulcus were miss-assigned in the modularity index algorithm. Table A.2 (in Annex A) gives details of the algorithm input and output. In practice, it is accepted that a Q value of above 0.3 is a good indicator of the existence of significant modules in a network. To estimate the confidence interval of the Q values for the data set, repeated calculations against 100 CT matrices generated on surrogate datasets was performed. Each of the 100 surrogate CT and SA matrices were generated by randomly drawing 213 subjects from the three study cohorts and calculating Q values on the correlation matrix obtained for CT and SA. The values of Q are shown in Figure 15, which is a plot of the distribution of the modularity index Q in regional CT networks generated on the 100 surrogate data sets. The central line indicates the mean value, and the upper and lower lines indicate 1.5 standard deviations away from the mean (Q = 0.36 ± 0.02), i.e., a random where Q values are similar to that of a random graph. For the study groups the following values were obtained: *Q_{HE}* = 0.49, *Q_{bvFTD}* = 0.49, and *Q_{AD} =* 0.45 for 'positive' and *Q_{HE}* = 0.39, *Q_{bvFTD}* = 0.28, and *Q_{AD}* = 0.29 for 'negative' sub-networks which indicate non-random modular topological organisation in the CT/SA correlation network frontal, temporal, parietal, and occipital divisions.

It can therefore be concluded that the cortical lobes as conventionally described correspond to non-random modules in the CT network.

### Mean correlation strength of the CT and SA networks

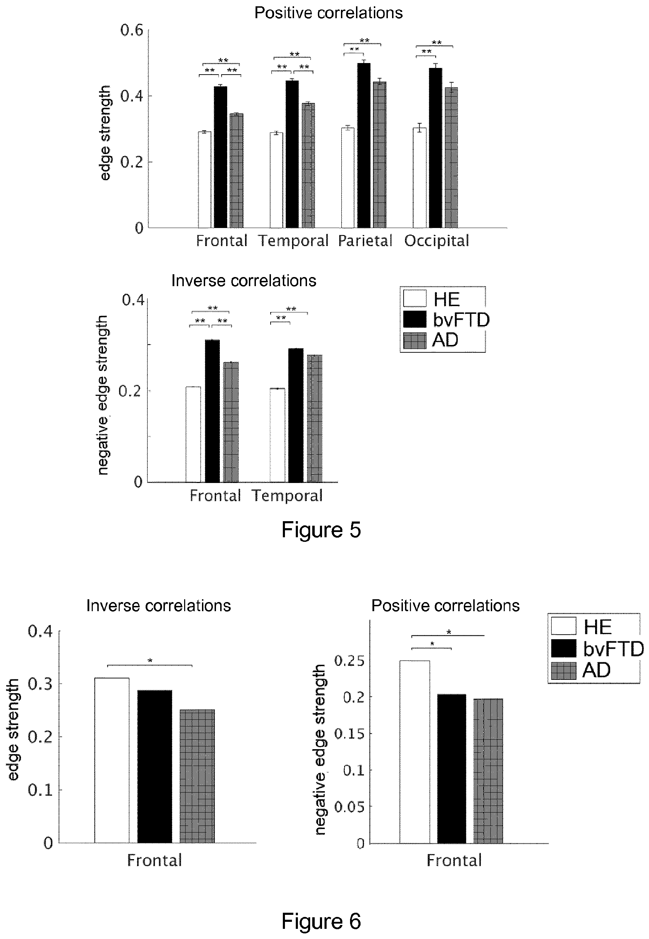

Figure 5 shows the edge strengths of each cortical thickness correlation network averaged over brain lobes and compared between HE, bvFTD, and AD groups. Data are shown for the networks of positive (upper plot) and inverse (lower plot) correlations. Asterisks indicate significant differences between the three groups (*p < 0.05; ** *p* < 0.01). As can be seen in Figure 5, the mean correlation strength for CT showed significant differences between HE, bvFTD, and AD subjects in frontal, temporal, parietal, and occipital lobes (p < 10⁻⁴ for all tests). The mean correlation strength was higher in bvFTD and AD than in HE subjects in frontal, temporal, parietal, and occipital lobes (for all pair-wise comparisons p ≤ 0.003). The mean correlation strength was higher in bvFTD than in AD in frontal (p < 10⁻⁴) and temporal *(p* = 0.005) lobes.

The mean strength of networks of inverse correlations in the CT network also differed in frontal and temporal lobes, see Figure 5 lower plot. Again, both bvFTD and AD groups showed higher mean correlation strengths than the HE group in frontal and temporal lobes *(p* ≤ 0.03 in all tests), and the bvFTD group had higher mean inverse correlation strengths than the AD group in frontal lobe (p = 0.003).

Figure 6 shows the edge strengths of each surface are a correlation network averaged over the frontal brain lobe and compared between HE, bvFTD, and AD groups. Data are shown for the networks of positive (right plot) and inverse (left plot) correlations. Asterisks indicate significant differences between the three groups (* *p* < 0.05; ** *p* < 0.01). The plots show significant differences in the mean correlation strength across the SA network. The diagnostic groups differed only in the frontal lobe where the AD group had a lower mean correlation strength than the HE group (p = 0.03). Similarly, inverse SA network correlations differed significantly in the frontal lobe with lower mean correlation strength in the bvFTD and

AD groups when compared with the HE group (p ≤ 0.02 in both tests). This is due to a larger number of correlations with a broader frequency distribution in strength found in disease compared with sparser networks having a narrower frequency distribution in healthy elderly subjects (see Figure 14).

### Nodal measures in the CT network

### Node degree

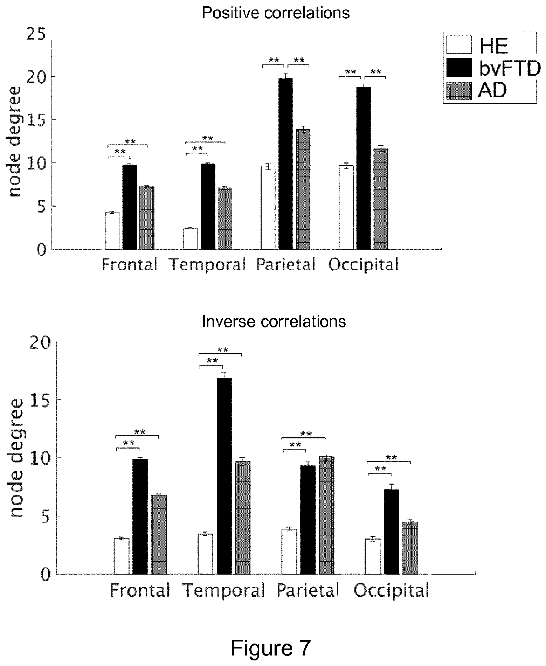

Node degree, which quantifies the mean number of significant positive correlations per node is shown averaged over frontal, temporal, parietal, and occipital lobes for the CT network in Figure 7. The node degree is compared across the HE, bvFTD, and AD groups. Data are shown for the networks of positive (upper plot) and inverse (lower plot) correlations. Asterisks indicate significant differences between the three groups (* *p* < 0.05; ** *p* < 0.01).

There were significant differences between groups in frontal, temporal, parietal, and occipital lobes (p ≤ 10⁻⁴ in all tests). Both bvFTD and AD subjects had higher node degree in frontal and temporal lobes (p < 0.006 for all tests) compared with HE subjects. The bvFTD group had notably higher node degree in parietal and occipital lobes than the AD group (p ≤ 0.02 for all tests). A similar pattern was found for the number of inverse correlations in the CT network in frontal, temporal, parietal, and occipital lobes (p ≤ 0.02 in all tests). These differences were driven by a larger number of significant inverse correlations in bvFTD and AD than in the HE group across all four lobes (p < 0.01 in all test). None of the differences between bvFTD and AD groups was significant.

### Node between-lobes participation index

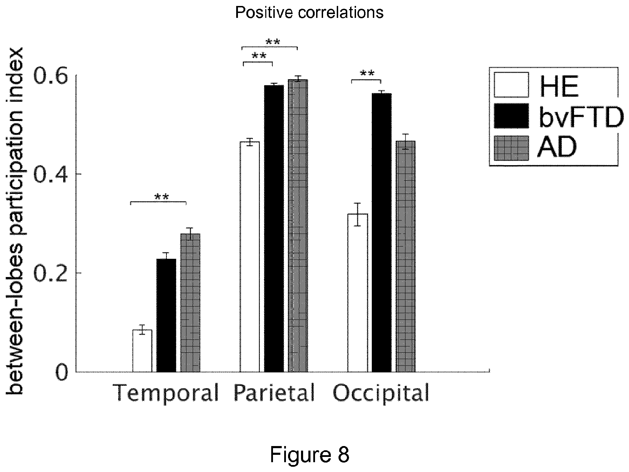

Group differences were found in the node between-lobes participation index for CT. The index measures the extent of significant positive correlation with nodes in different lobes. This was significant for lobes located in the temporal, parietal, and occipital lobes (p ≤ 0.03 for all tests). The differences reflect higher index values relative to the HE group in the parietal (p < 0.003 in both groups), temporal *p* = 0.01 in AD) and occipital (p = 0.002 in bvFTD) lobes. This is shown in Figure 8, where node between-lobes participation index of the cortical thickness correlation network averaged over brain lobes is compared across HE, bvFTD, and AD groups. Data is shown for the positive correlations only in the plot. Asterisks indicate significant differences between the groups (* *p* < 0.05; ** *p* < 0.01). None of the between-lobes participation index comparisons was significant different in any lobe for the inverse correlations in the CT network.

### Nodal measures in the SA network

### Node degree

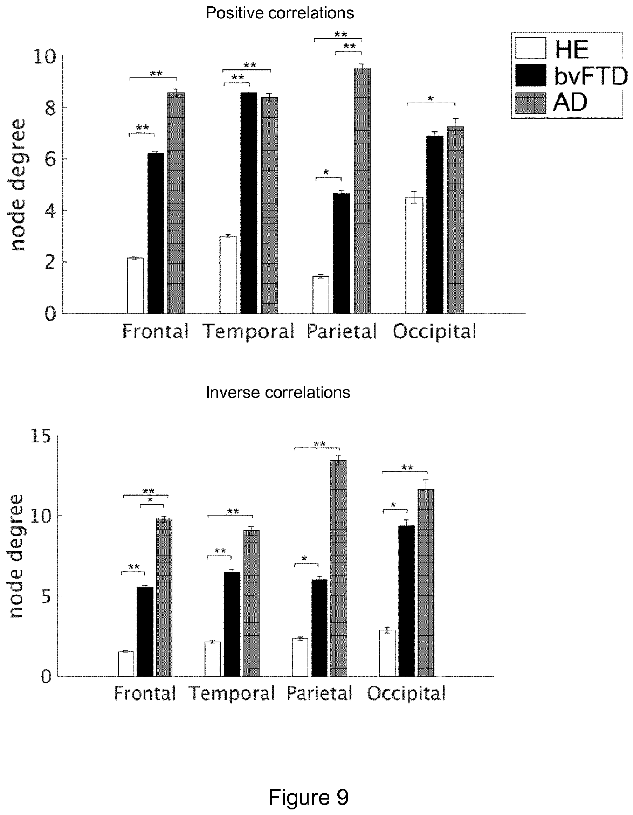

Node degree values in the SA network are shown for frontal, temporal, parietal, and occipital lobes in Figure 9. In the figure, node degree of the surface area correlation network is averaged over brain lobes and compared across HE, bvFTD, and AD groups. Data are shown for the networks of positive (upper plot) and inverse (lower plot) correlations. Asterisks indicate significant differences between the three groups (* *p* < 0.05; ** *p* < 0.01).

Positive correlations differed between diagnostic groups in frontal, temporal, parietal, and occipital lobes (p ≤ 0.03). As with the CT network, both bvFTD and AD groups had higher SA node degree than the HE group in frontal, temporal, and parietal lobes (p < 10⁻⁴ in all tests). For the occipital lobe the only difference which was significant was between the AD and HE groups (p = 0.04). In contrast to the CT network, the node degree in the parietal lobe was also significantly higher in AD than in bvFTD (p = 0.004).

The inverse correlation SA network also showed significant group differences in frontal, temporal, parietal, and occipital lobes (p ≤ 0.001 in all tests). Again, both bvFTD and AD groups had higher node degrees than the HE group in all four lobes (p < 0.001 for all tests). In contrast to the CT inverse correlation network, the AD group had higher node degree than the bvFTD group in the frontal (p = 0.02) and parietal (p = 0.01) lobes.

### Node between-lobes participation index

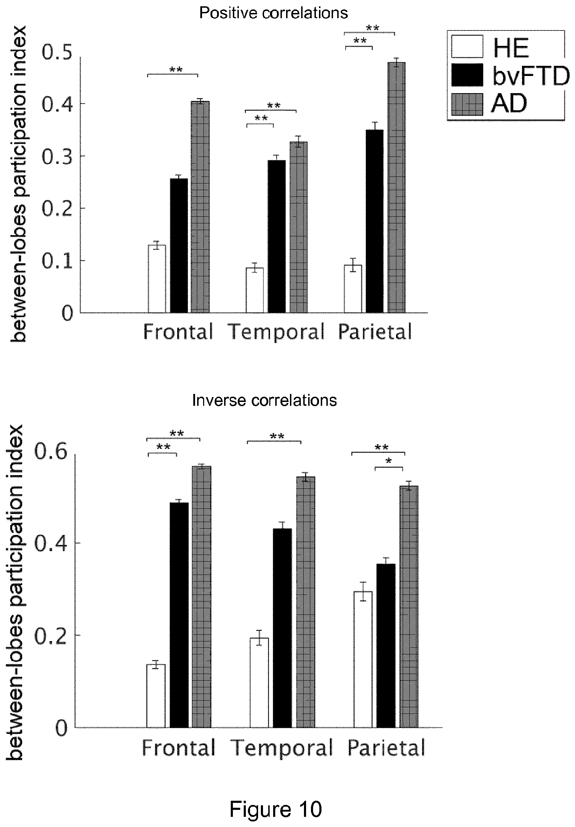

Figure 10 shows group differences in the nodal between-lobes participation index for the SA network organisation. The figure shows node between-lobes participation indexes averaged over brain lobes and compared across HE, bvFTD, and AD groups. Data are shown for the networks of positive (upper plot) and inverse (lower plot) correlations. Asterisks indicate significant differences between the three groups (* *p* < 0.05; ** *p* < 0.01).

Both bvFTD and AD groups had higher index values than the HE group for the positive SA correlation network in all four lobes (p < 10⁻⁴). In contrast to the CT correlation network, the inverse SA correlation network also showed significant differences in frontal and parietal lobes (p ≤ 0.04 for both patient groups) and in temporal lobe for the AD group (p < 0.001) relative to the HE group.

### Hubs of the structural correlation networks

### CT Network hubs

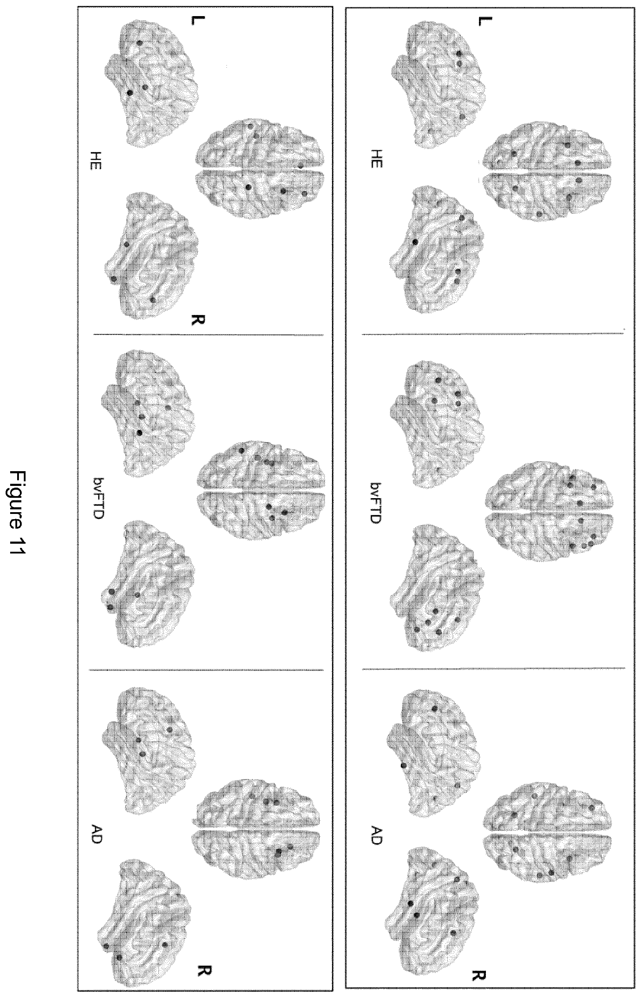

There are four possible combinations of mean values of between-lobes participation index (p high/low) and with-lobe z-score (high/low). Here, only the case of high between-lobes index and high within-lobe z-score are considered in order to focus on nodes high hub-like characteristics. Tables A.4-A.6 (see Annex A) provide data for the global and provincial network hubs. The remaining two combinations were examined, but were uninformative. The number and distribution of network hubs within high p and high z values in the positive CT correlation network differed between study groups. In the HE subjects, hubs were distributed across the whole cortex; each lobe had at least one hub, with four hubs in the frontal lobe. The re-organisation of hub topology occurred differently in the two disease groups. This is shown in Figure 11, upper panel, which is a visualisation in brain space of the hubs of the cortical thickness network. In bvFTD the number of hubs in the frontal lobe increased from 4 to 9, decreased from 2 to 1 in the occipital lobe, and vanished completely in parietal and temporal lobes. By contrast, hubs were distributed across all four lobes in AD almost equally. The number of hubs in the frontal lobe decreased (2 vs 4), whereas the number increased relative to the HE group in the temporal and occipital lobes (1 vs 3 and 2 vs 3, respectively). A full list of nodes which have hub-like properties in the CT network is provided, along with lobar positions, in Table A.4 (see Annex A).

Nodes with hub-like properties in the inverse correlation CT matrix were present exclusively in frontal and temporal lobes in all three groups and their topological distribution differed between the groups. See Figure 11 lower panel, and table A.4 in Annex A.

### SA network hubs

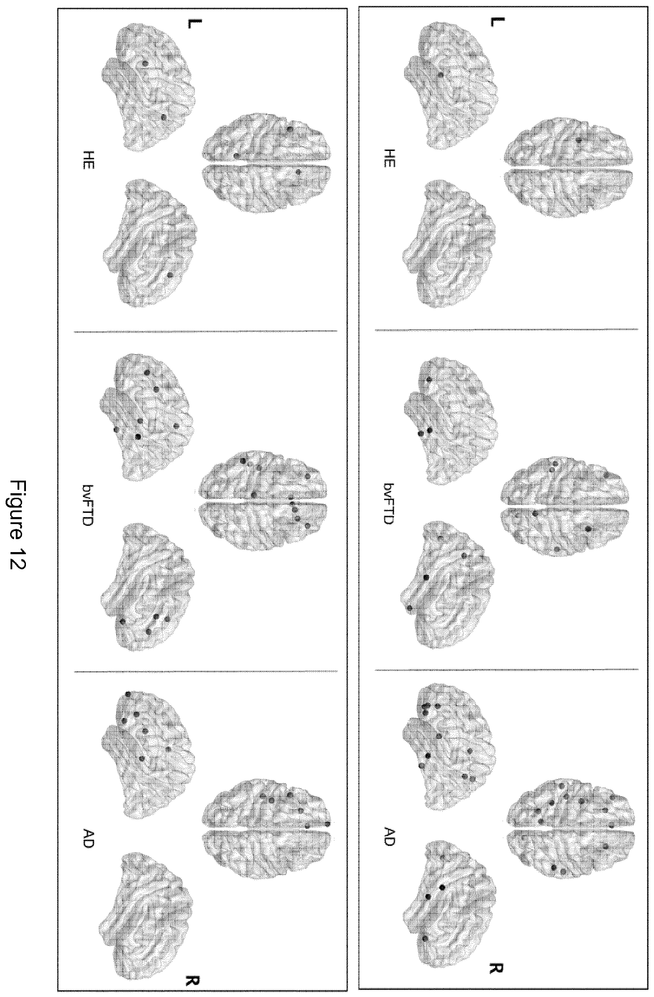

Hubs in the positive correlation SA network are shown in Figure 12 upper panel. Table A.5 (see Annex A) provides a list of nodes and lobar locations classified according to between-lobe participation index and within-lobe z-score. A visual comparison of hub topology between groups show that the left hemisphere had more nodes with hub-like properties in all diagnostic groups. However, HE subjects had only one SA hub (left insula), whereas both disease groups had more hubs in each lobe. The AD group had twice as many SA hubs compared to bvFTD (14 vs 7). Surprisingly, the bvFTD group had more SA hubs in the temporal than in the frontal lobe (4 vs 1), while AD subject had more frontal than temporal hubs (6 vs 4). AD subjects had 3 hubs in the parietal lobe compared with 1 in the bvFTD subjects.

Hubs in the inverse correlation SA network were present in either frontal or temporal lobe only in all three groups. However, the HE group had one hub in the parietal (precuneus) and bvFTD had two (inferiorparietal and paracentral) (see Table A.5 in Annex A). Interestingly, most of the inverse correlation SA hubs in AD were found in the frontal lobe.

### Cortical thickness - cortical surface area coupling topology

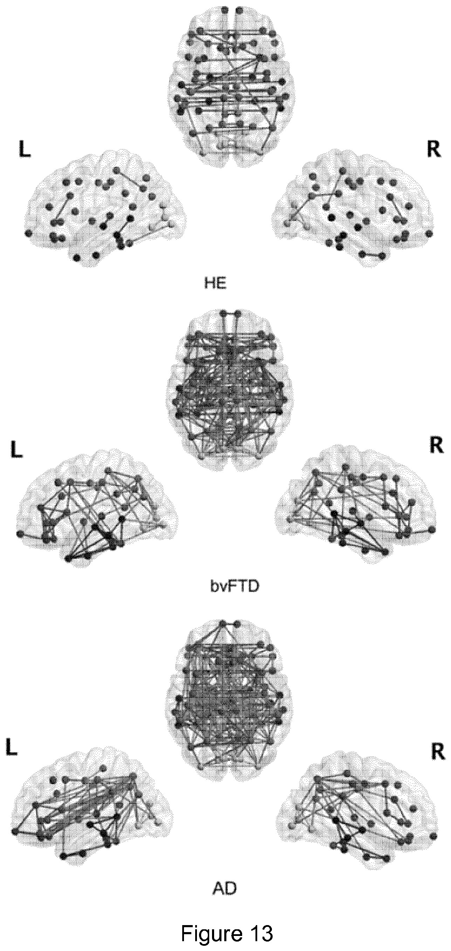

The coupling strength between CT and SA nodes was calculated by element-wise multiplication of corresponding CT and SA correlation matrices. Figure 13 shows CT/SA coupling strength visualised in brain space. It can be seen that, in HE subjects, pairs of inter-hemispheric homologues show coupled CT/SA correlation. By contrast, CT/SA coupling in AD and bvFTD groups are very similar to each other and different from the HE group. Both the bvFTD and AD groups showed more coupling between non-homologous nodes in the same and contra-lateral hemispheres. The inter-lobar correlations were also strikingly different between the bvFTD and AD groups. In the bvFTD group, most of the inter-lobar CT/SA correlations were due to fronto-temporal interactions. In AD, most of the inter-lobar CT/SA coupling was due to fronto-parietal interactions. A list of hubs of CT/SA coupling topology is given in Table A.6 in Annex A.

### Discussion

Baseline structural correlation networks in subjects diagnosed clinically with either bvFTD or AD in three large global clinical trials have been examined, and compared with healthy elderly subjects in a well-characterised birth cohort. For each group, networks were constructed from the partial correlations between 68 x 68 pairs of cortical surface regions (nodes) in terms of their thickness and surface area. The approach adopted has permitted a systematic analysis if both positive and inverse network correlations in the three clinical contexts. The methods and data discussed herein represent the first systematic comparative analysis of cortical thickness and surface area in a large population of subjects. Since the numbers needed to be comparable in the three groups, the overall study size was determined by the number of bvFTD subjects available. As this is a rare disease, it was necessary for the bvFTD component of the study to be global, with patients entering from 70 trial sites in 13 countries. With 213 patients included in the study, this represents the largest set of MRI scan data in bvFTD subjects available thus far. In order to match this, 213 patients were drawn randomly from a much larger group of 1131 AD patients coming from 116 sites in 12 countries for study TRx-237-005 and 128 sites in 16 countries for study TRx-237-015 (accessible for example from the US National Library of Medicine). The 202 normal elderly subjects come from a well-characterised birth cohort that has been studied longitudinally. The findings reported are therefore robust and can be considered representative of international populations meeting accepted diagnostic criteria.

### Modularity of networks by lobes

It has been shown that the structural correlations in frontal, temporal, parietal, and occipital divisions of the cortical surface are inherently modular for both the cortical thickness and surface area networks. That is, the results confirm that the standard lobar divisions of the cortex share common network modularity attributes, such that they differ from what would be expected in a comparable random network. Modules of highly clustered networks confer so called 'small-world' network properties and are thought to provide an optimal balance between local specialisation and global integration. The results from healthy elderly subjects are comparable with prior work in a smaller and younger healthy group revealing an underlying modular architecture in the regional thickness correlation network. The results also indicate that intrinsic lobe-wise modularity persists in both bvFTD and AD, indicating that the overall lobar architecture of the networks is preserved in the presence of neurodegenerative change. As discussed further below, this contrasts with the hub-like organisation of the networks which changes in a disease-specific manner.

### Similarities and differences between AD and bvFTD relative to healthy elderly subjects

The morphological correlation networks for both patients group (bvFTD and AD) were found to differ from the corresponding network for healthy elderly subjects in highly significant ways. Both groups showed a striking increase in the overall correlation strength in thickness and surface area networks compared with healthy elderly subjects. The effect was more pronounced in the cortical thickness network in all lobes for both positive and inverse correlations. This contrasts with a significantly lower correlation strength relative to normal for surface area in frontal lobe in AD and a directionally similar difference in bvFTD. This may be due to a larger number of correlations with a broad frequency distribution in disease as compared with sparser networks having a narrower frequency distribution in healthy elderly subjects. In addition to increased overall correlation strength, the number of within-lobe positive and inverse correlations as measured by node degree was higher in all lobes in both dementia groups than in healthy elderly controls. The number of between-lobe positive correlations in thickness as measured by the between-lobe participation index was also higher in all lobes. The number of within-lobe and between-lobe positive surface area correlations was also greater in both bvFTD and AD than in healthy elderly subjects in frontal, temporal, and parietal lobes. Both disease groups also differed from healthy elderly subjects in terms of the correlations in coupling between cortical thickness and surface area. Thus, both diseases are characterized by an overall increase in the strength and extent of structural correlation occurring both locally within lobes and globally between lobes.

The similarity between the two conditions in terms of the marked increase in overall strength and extent of structural correlation might appear to call into question the clinical distinctions between bvFTD and AD on which the classification of subjects in the study was based. Indeed, there were no differences between the two conditions in terms of overall cortical thickness and surface area. However, there were a number of important network differences between the two conditions. In the cortical thickness network, the overall positive correlation strength has been greater in bvFTD than in AD in frontal and temporal lobes, and the inverse correlation strength was also greater in bvFTD than in AD in the frontal lobe. The number of significant positive within-lobe correlations was higher in bvFTD than in AD in parietal and occipital lobes. Conversely, the number of positive and inverse within-lobe correlations was greater in AD than in bvFTD frontal and parietal lobes. Most of the inverse correlations in cortical thickness and surface area related to inter-hemispheric non-homologous fronto-temporal lobes in bvFTD and in fronto-parietal lobes in AD.

The hub-like organisation of the correlation networks also differed substantially in the two conditions. Network connector hubs are though to provide network integration, whilst provincial hubs provide network segregation. It has been proposed that hubs provide resilience to insult in neurodegenerative disorders. Alternatively, it has been suggested that the hubs represent loci of particular vulnerability. It is therefore of interest to study how the hubs change in the context of neurodegenerative disease. bvFTD was characterised by an increase in the number of cortical thickness hubs in frontal lobe and a reduction or elimination of hubs in temporal, parietal, and occipital lobes. By contrast, AD was characterised by hubs distributed in all lobes, a reduction in the number of hubs in frontal cortex, and an increase in hubs in temporal and occipital lobes compared with bvFTD. In the positive correlation network for surface area, AD subjects had twice as many hubs overall than bvFTD, and the topology of these hubs differed. Thus overall, AD is characterised by a much more distributed pattern of hubs in both the thickness and surface area degenerative networks than bvFTD. By contrast, the hub-like organisation is much more localised in bvFTD. It has been argued that bvFTD is a clinical syndrome with focal but heterogeneous atrophy centred around hubs. Identification of the insular region as one of the inverse network hubs (in both bvFTD and AD groups for the CT network) is consistent with the recent unexpected finding from diffusion MRI that there is an increase in hub-like fibre connectivity of the insula in bvFTD. The hubs of the healthy elderly group, on the other hand, were highly connected within and between lobes in a homologous fashion and were not otherwise linked to each other. The differences in hub-like organization between AD and bvFTD indicates differences in the hierarchy of nodal vulnerability and in the organisation of network adaptations compensating differently in the two conditions. Thus, unlike lobar modularity, which is preserved in neurodegenerative disease, a constant hub-like organisation is not preserved, implying that pre-existing hubs are not an intrinsic structural property of cortical network organisation.

Although AD is also characterised by changes in cortical thickness, these are on the whole less marked than in bvFTD, whereas the changes in surface area are more prominent in AD, suggesting co-ordinated changes in numbers of adjacent affected columns. These differences would be consistent with the pathology of bvFTD affecting interneurons and astrocytes which have more localised links. The predominance of surface area correlations in AD would be consistent with the pathology affecting primarily long-tract cortico-cortical projection systems mediated by the principal cells. bvFTD differs in a number of important respects from AD: there is no cholinergic deficit in bvFTD, there is no treatment benefit from treatment with either acetylcholinesterase inhibiters or memantine, bvFTD is characterised by prominent astrocytic pathology, neurons affected in the neocortex are predominantly spiny interneurons in layers II and VI (pyramidal cells in layers III and V are predominantly affected in AD) and dentate gyrus of hippocampus (neurons affected in AD are in CA 1-4 and not dentate gyrus) and bvFTD is characterised by increased glutamate levels in the neocortex but AD is not. However, none of these conditions provide a simple explanation for the different distribution patterns of the correlated structural changes described herein.

### Global character and significant of network changes in dementia

The overall picture which emerges from the two disease groups studied is that network architecture is changed in a co-ordinated fashion throughout the whole brain as regards both positive and inverse correlations. This is surprising, given that the neurodegenerative processes in these two conditions are generally considered to be anatomically restricted, to frontal and temporal lobes in the case of bvFTD and to temporal and parietal lobes in AD. Rather, the network analysis suggests that there are changed in cortical thickness and surface area networks in both conditions that affect all lobes in a global manner, but that there are differences in the anatomical topology of the changes. Both Tau and TDP-43 aggregation pathology is known to spread in prion-like fashion, whereby pathology in an affected neuronal population can initial pathology in a connected, but previously unaffected neuronal population. The positive correlations could therefore reflect in part the spread of pathology in existing normal networks whereby existing functional networks are affected or spared together. Alternatively, such correlations might express functional dependencies, such that loss of function in one member of a partnership results in a parallel loss of function in a partner normally synchronized functionally with an affected node. This interpretation would be consistent with previous work on cortical thickness correlations in healthy adults, where positive correlations were found to converge with diffusion-based axonal connections.

The work discussed herein highlights for the first time the significance of inverse correlation networks. It should be noted that because the inverse correlations seen in both neurodegenerative disorders reflect primarily inter-lobar non-homologous associations, they would not have been detected using only a lobe-based approach to the analysis. It is particularly the appearance of these non-homologous inverse inter-lobar correlations and their increased strength that represents the clearest overall difference between neurodegenerative disease and normal aging. By contrast, the normal aging brain is characterised by substantially weaker homologous positive correlations. An attractive hypothesis is that, as certain nodes become functionally impaired, other still unaffected nodes compensate, accentuating non-homologous associations in disease. This would imply that the major reorganisation in structural network observed may be partly adaptive in character. Structural plasticity has been demonstrated in other contexts, and functional compensation is known to occur in focal disease.

The work discussed herein represents a first comparative study of correlated structural network abnormalities in bvFTD and AD relative to healthy aging. These correlations arise from both positive and inversely linked changes in cortical thickness and surface area in the two disease conditions, which are quite different from those seen in normal elderly subjects. The changes seen in disease are global in character and are not restricted to fronto-temporal and temporo-parietal lobes respectively in bvFTD and AD. Rather, they appear to represent structural adaptations to neurodegeneration which differ in the two conditions. Further, all of the correlation networks showed a quite distinctive hub-like organisation which differs both from normal and between the two forms of dementia. Unlike lobar organisation of networks, which remains constant in disease, hub-like organisation varies with the underlying pathology. This implies that hub-like organisation is not a fixed feature of the brain and attempts to explain disease in terms of hubs may be inadequate. The differences between AD and bvFTD documented confirm that the clinical differences in the two dementia populations correspond to systematic differences in the underlying network structure of the cortex. The topological differences in thickness and surface-area hub-like organisation, as well as the underlying positive and inverse correlation networks, may provide a basis for development of analytical tools to aid in the differential diagnosis in the two conditions, which can be difficult to distinguish by purely clinical criteria.

### Use of correlation matrices in determining patient group response to neuropharmacological intervention

The methods discussed above have been used to determine patient group response to neuropharmacological intervention.

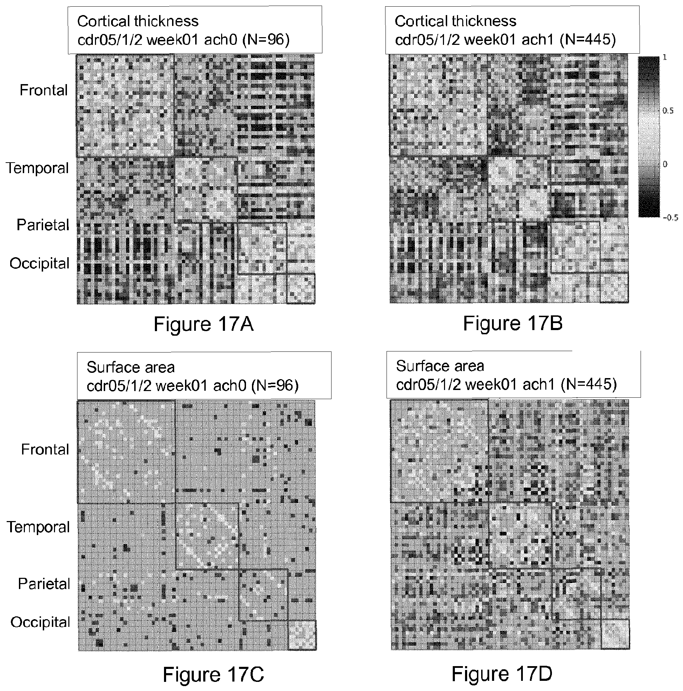

Figures 17A - 17D depict correlation matrices for two patient groups, those being treated with symptomatic AD drugs (cholinesterase inhibitor and/or memantine; ach1 in the figure captions) and those who are not (ach0 in the figure captions). The subjects ranged in clinical dementia rating (CDR) score from 0.5, 1, or 2. Figure 17A is a cortical thickness correlation matrix at baseline (i.e. week 0) for 96 subjects diagnosed with AD who are not taking symptomatic treatment(s). In contrast, Figure 17B is a cortical thickness correlation matrix at base link for 445 subjects diagnosed with AD who are taking symptomatic treatment(s). Figure 17C is a surface area correlation matrix at base line for 96 subjects diagnosed with AD who are not taking symptomatic treatment(s), and Figure 17D is a surface area correlation matrix at base line for 445 subjects diagnosed with AD who are taking symptomatic treatment(s).

As can be seen from Figures 17A - 17D, symptomatic treatment(s) for AD induce a significant increase in inter-lobar non-homologous inverse correlation networks (blue in Figures 17B and 17D) as compared to untreated patients (Figures 17A and 17C). This is particularly noticeable for surface area networks.

These connections represent inverse correlations whereby a decrease in the volume or surface area of an affected area in a particular node (typically located in the posterior parts of the brain) is correlated in a statistically significant manner with a linked node where there is a corresponding increase in the volume or surface area. As discussed above, the presence of these non-homologous inverse correlations is indicative of neurodegenerative disease and most likely represent frontal compensation for posterior dysfunction arising from pathology. Symptomatic AD treatments induce an increase in these non-homologous compensatory linkages.

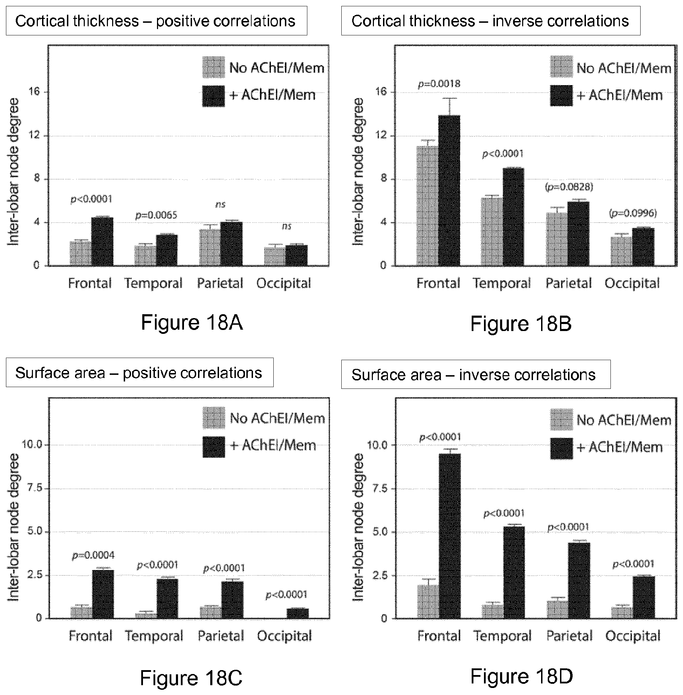

Figures 18A - 18D are plots of non-homologous inter-lobar node degree (as discussed above) for, respectively, cortical thickness - positive correlations, cortical thickness - inverse correlations, surface area - positive correlations, and surface area - inverse correlations. As can be seen from these plots, the numbers of significant non-homologous inter-lobar compensatory inverse correlations is greatly increased by symptomatic AD treatments.

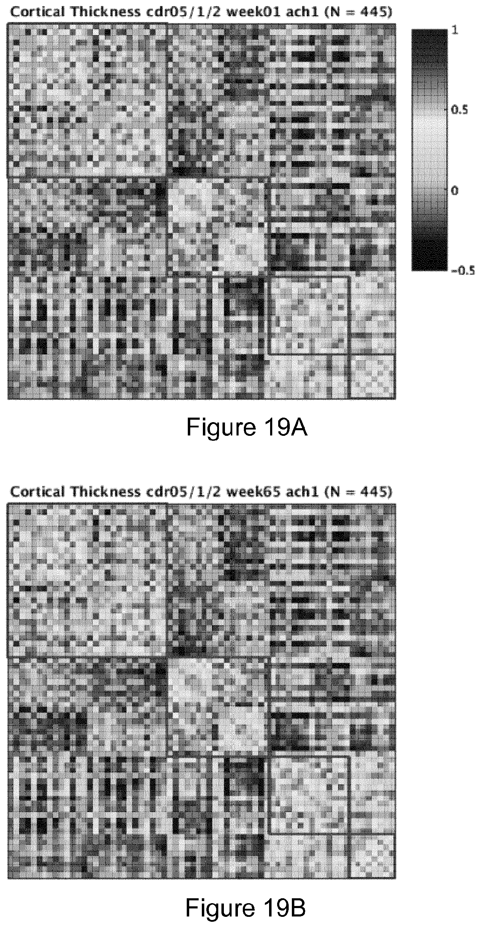

Figures 19A and 19B show cortical thickness correlation matrices based on structural neurological data which is temporally separated. Figure 19A is a cortical thickness correlation matrix at week 0 (i.e. at baseline) for a group of 445 AD diagnosed patients who are being treated with symptomatic AD treatments. Figure 19B is a cortical thickness correlation matrix at week 65 for the same group of 445 AD diagnosed patients. During the intervening period, the group have also been treated with leuco-methylthioninium mesylate (LMTM; USAN name: hydromethylthionine mesylate), a tau aggregation inhibitor, at a dosage of 8mg/day (4 mg given twice daily here, and in what follows). As can be seen, LMTM has a minimal effect on structural correlation networks in patients receiving symptomatic treatments for AD.

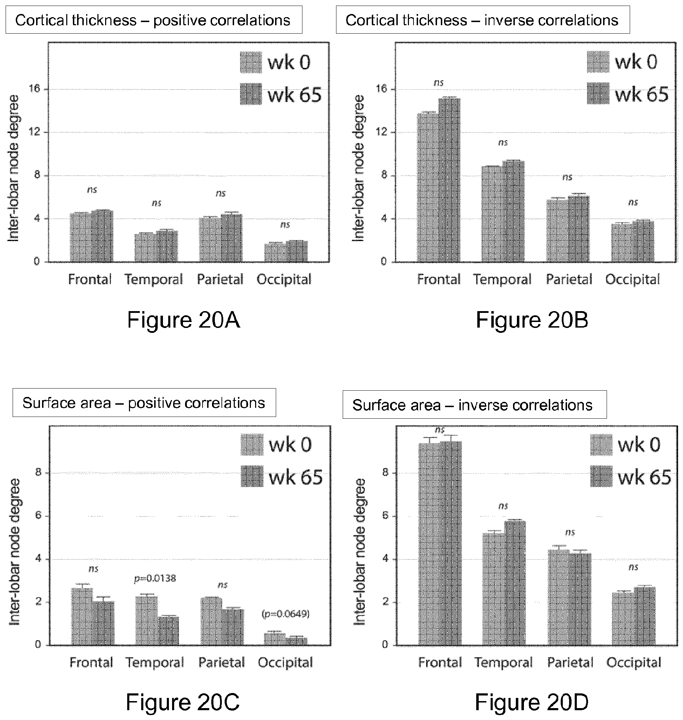

Figures 20A - 20D are plots of non-homologous inter-lobar node degree as compared between week 0 and week 65 in the ach1 group (taking symptomatic AD treatments concomitantly) for, respectively, cortical thickness - positive correlations, cortical thickness - inverse correlations, surface area - positive correlations, and surface area - inverse correlations. As can be seen, there is a minimal overall effect of LMTM as an add-on on brain network correlation structures over 65 weeks. It is important to note that this is a within-cohort analysis whereby patients at baseline serve as their own controls for the changes occurring after 65 weeks of treatment with LMTM.

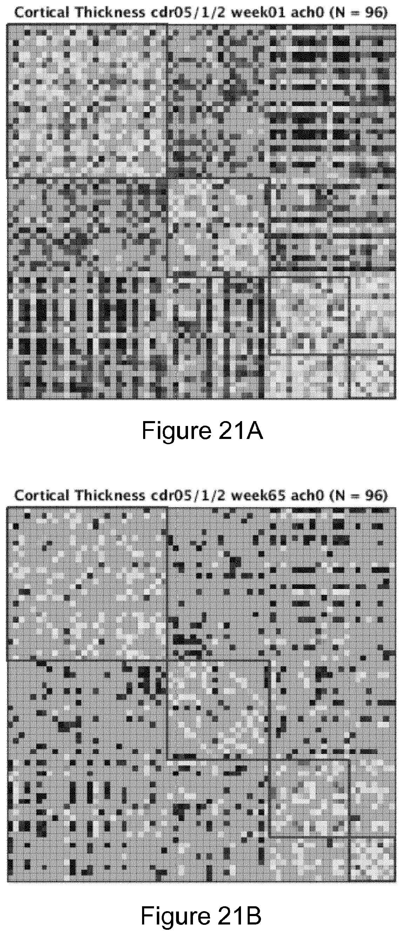

Figures 21A and 21B show cortical thickness correlation matrices based on structural neurological data which is temporally separated. Figure 21A is a cortical thickness correlation matrix at week 0 (i.e. at baseline) for a group of 96 AD diagnosed patients who took LMTM as a monotherapy at a dosage of 8mg/day. Figure 21B is a cortical thickness correlation matrix at week 65 for the same group of 96 AD diagnosed patients. The 96 patients in this cohort were not receiving symptomatic AD treatment(s) in combination with LMTM. As can be seen, LMTM as a monotherapy produces a major reduction in thickness correlations, both within-lobe (positive) and between-lobe compensatory (inverse) correlations. This is a within-cohort analysis whereby patients at baseline serve as their own controls for the changes occurring after 65 weeks of treatment with LMTM.

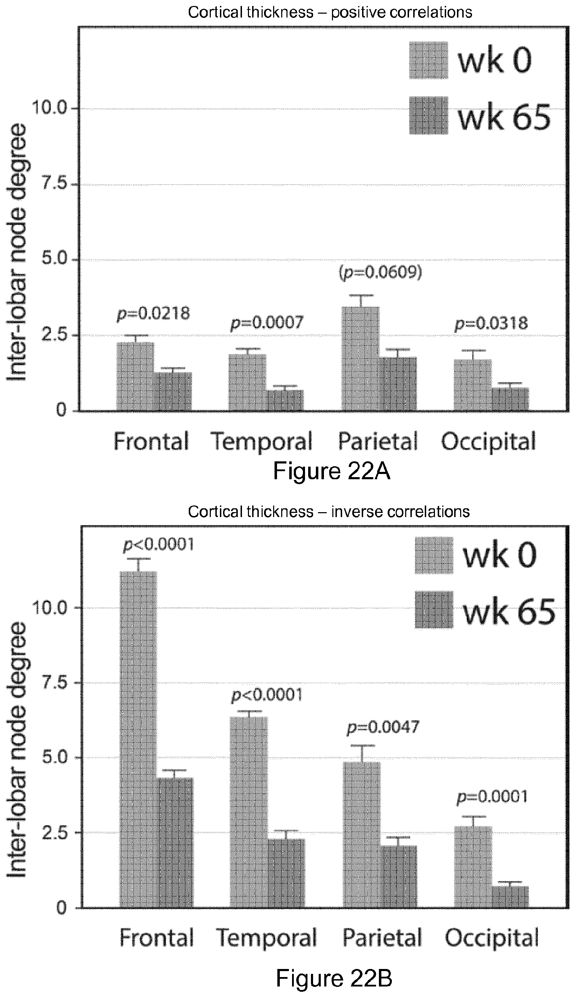

Figures 22A and 22B are plots of inter-lobar node degree as compared between week 0 and week 65 in the ach0 group for cortical thickness - positive correlations and cortical thickness - inverse correlations. The plots indicate highly significant effects of 8 mg/day LMTM as a monotherapy on the number of inter-lobar correlations in the AD group. A significant reduction in the number of positive and inverse non-homologous cortical thickness correlations is seen after 65 weeks. This is likely to be due to normalisation of neuronal function in the posterior parts of the brain whereby LMTM reduces the pathology and reduces the neuronal dysfunction arising from pathology, thereby reducing the need for compensatory input form the unaffected or less affected frontal regions of the brain.

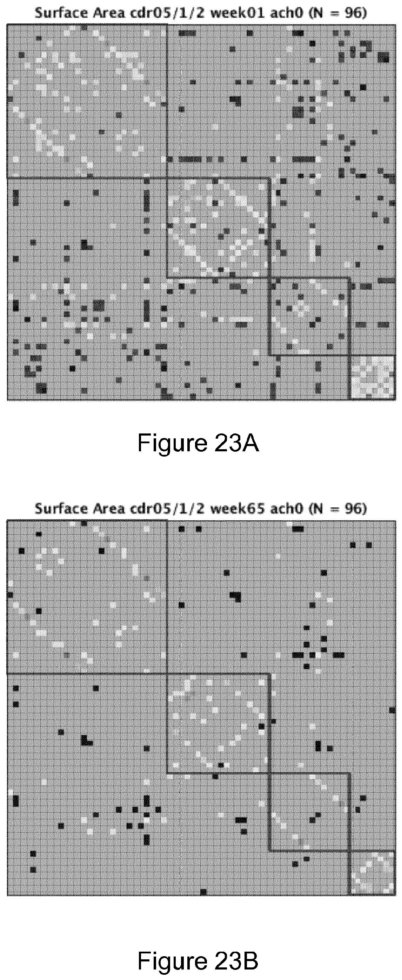

Figures 23A and 23B show surface area thickness correlation matrices based on structural neurological data which is temporally separated. Figure 23A is a surface area correlation matrix at week 0 (i.e. baseline) for a group of 96 AD diagnosed patients who went on to take LMTM as a monotherapy at a dosage of 8mg/day. Figure 23B is a surface area correlation matrix at week 65 for the same group of 96 AD diagnosed patients. The 96 patients in this cohort were not taking concomitant symptomatic AD treatment(s). As can be seen, LMTM as a monotherapy produces significant reductions in surface area correlations, both within-lobe (positive) and between-lobe compensatory (inverse) correlations. This is a within-cohort analysis whereby patients at baseline serve as their own controls for the changes occurring after 65 weeks of treatment with LMTM.

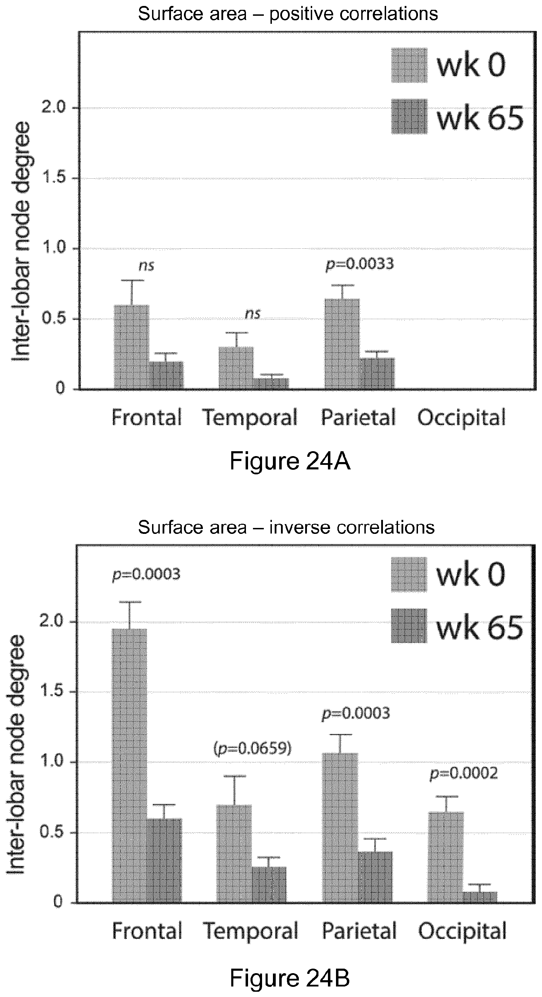

Figures 24A and 24B are plots of non-homologous inter-lobar node degree as compared between week 0 and week 65 in the ach0 group for surface area - positive correlations and surface area - inverse correlations. The plots indicate significant effects of 8 mg/day LMTM as a monotherapy on the number of inter-lobar correlations in the AD group. Notably, there is a significant reduction in the number of positive and inverse/compensatory surface area correlations after 65 weeks.

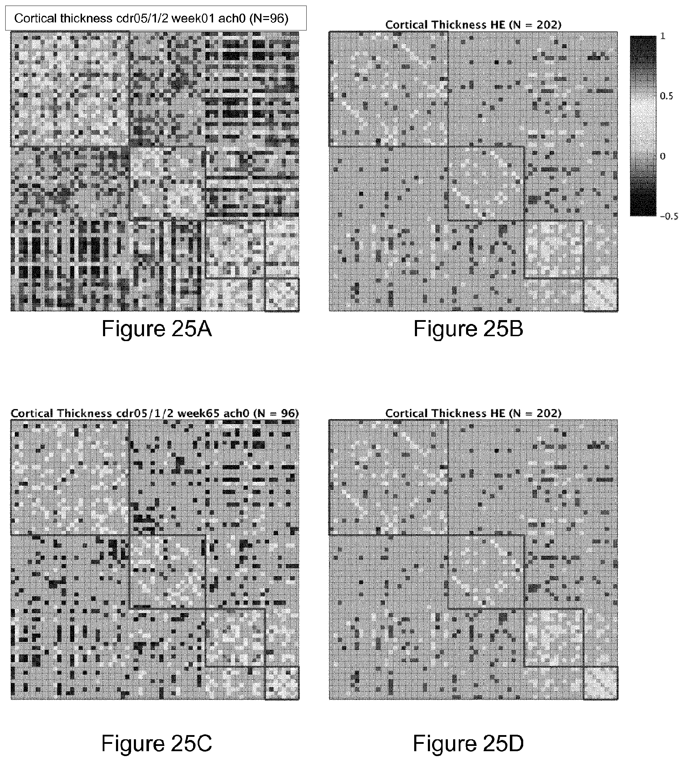

Figures 25A - 25D show cortical thickness correlation matrices compared between the 96 patient AD group (with CDR 0.5, 1, or 2) at baseline and week 65 as compared to the 202 subject healthy elderly control group. As can be seen, LMTM at 8mg/day as monotherapy brings the cortical thickness networks closer to normal.

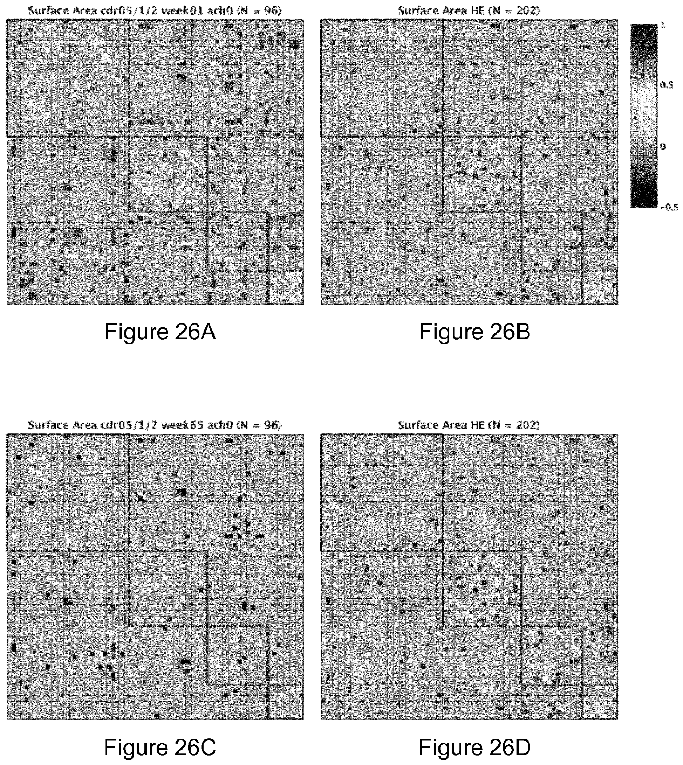

Figures 26A - 26D show surface area correlation matrices compared between the 96 patient AD group (with CDR 0.5, 1, or 2) at baseline and week 65 as compared to the 202 subject healthy elderly control group. As can be seen, LMTM at 8mg/day as monotherapy normalises surface area networks.

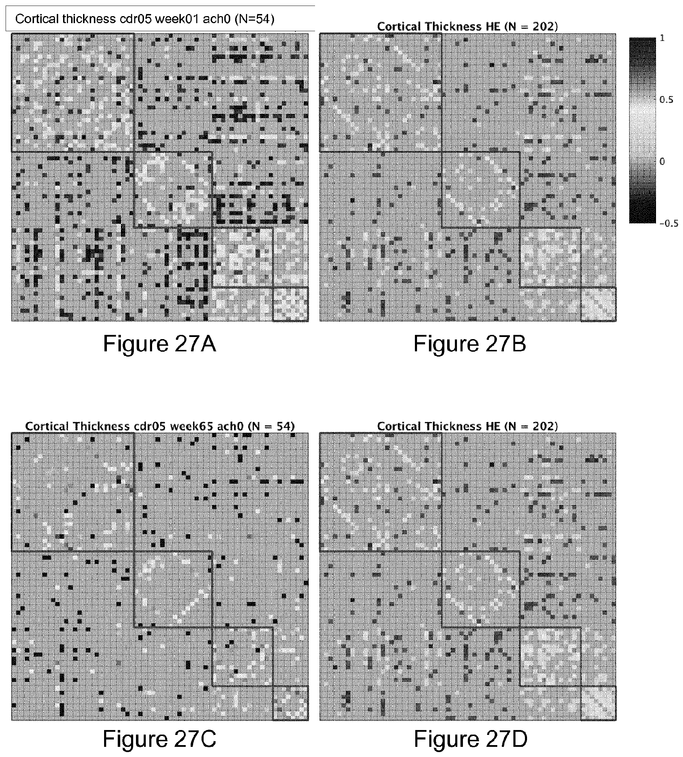

Figures 27A - 27D show cortical thickness correlation matrices compared between the 54 patient AD group with CDR of 0.5 only at baseline and week 65 as compared to the 202 subject healthy elderly control group. As can be seen, LMTM at 8mg/day as monotherapy reduces the number of inverse/compensatory non-homologous correlations to become equivalent to the normal elderly controls.

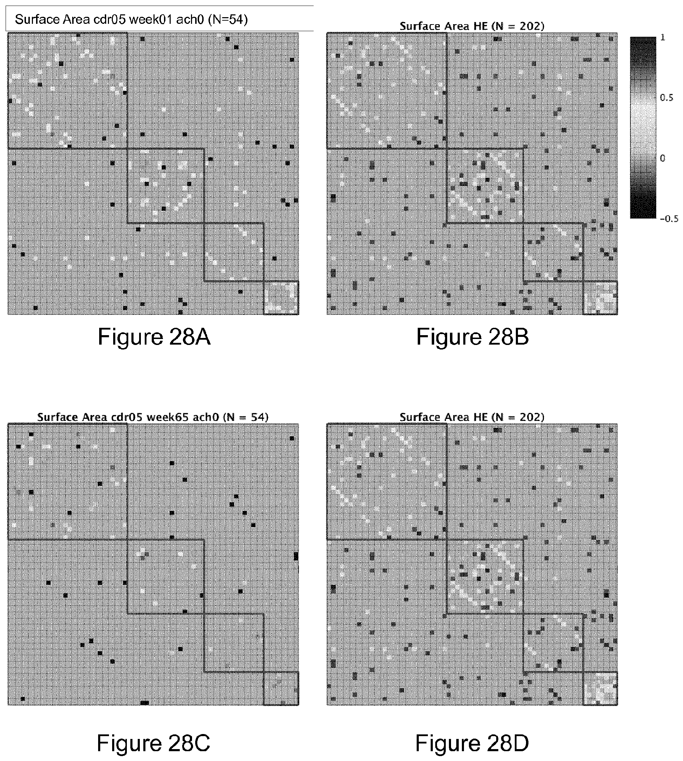

Figures 28A - 28D show surface area correlation matrices compared between the 54 patient AD group with CDR of 0.5 only at baseline and week 65 as compared to the 202 subject healthy elderly control group. As can be seen, LMTM at 8mg/day as monotherapy reduces the number of inverse/compensatory non-homologous correlations to either equivalent to or lower than normal elderly controls.

In summary, the structural correlation network analysis discussed above reveals the emergence of highly abnormal inverse non-homologous inter-lobar correlations in AD and bvFTD. It is hypothesised that these represent compensatory input from frontal brain regions unaffected or less affected by disease. Symptomatic treatments and LMTM act in fundamentally different ways in AD in terms of the structural correlation network. Symptomatic treatments induce substantial increases in the compensatory networks. LMTM as monotherapy reduces the need for these compensatory networks by reducing the primary pathology thereby permitting affected neurons to function more normally. These results confirm that the abnormal inverse non-homologous correlations seen in neurodegenerative diseases such as AD are adaptive in character, since they can be reversed or attenuated by disease-modifying treatment, but not by symptomatic AD treatments. The effects are seen in within-cohort before/after analysis in which subjects at baseline serve as their own controls for the changes occurring after receiving LMTM treatment at 8 mg/day as monotherapy for 65 weeks. These analyses are far more sensitive to treatment effects than crude whole brain or lobar volume analyses. Further, as will be discussed below, the results seen in terms of structural correlation networks are consistent with the functional effects seen by re-normalised partial directed coherence electroencephalography analysis techniques.

### Structure/function correlation using electroencephalography (EEG)

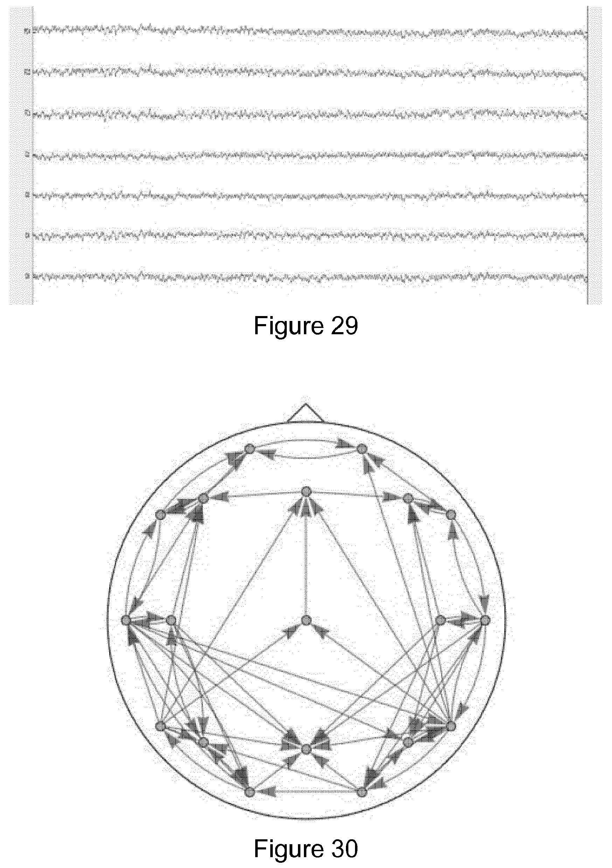

Re-normalised partial directed coherence (rPDC) network approaches to EEG data allow an indication of the direction and strength of electrical activity within the brain to be investigated using a network approach. This is discussed, for example, in WO 2017/118733. Figure 29 shows an example of raw EEG data, and Figure 30 shows an example rPDC network resulting from the collected EEG data.

The resulting network, as shown in Figure 30, comprises a number of nodes indicative of approximate locations within a brain (the figure is drawn in a schematic fashion looking down on the head with the triangle at the top indicating the nose). The location of the nodes is determined by placement of the electrodes on the scalp surface that are used to obtain the EEG data such as that shown in Figure 29. The directed connections between the nodes indicate a flow of electrical activity from one node to another within the brain.

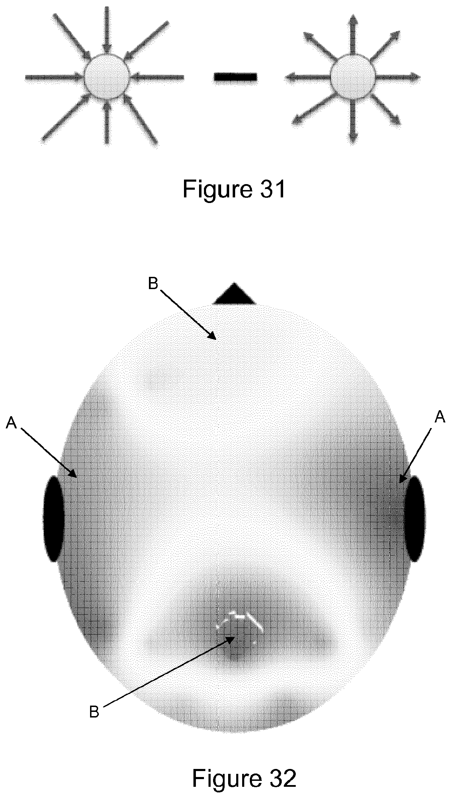

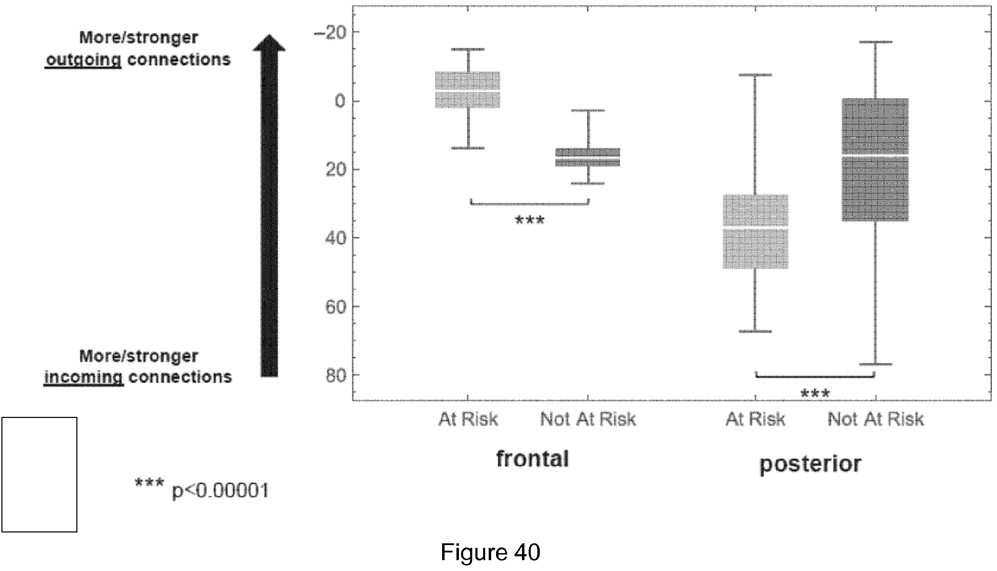

By counting the number of directed connections into and out of a given node and/or measuring their relative strength, it is possible to define whether a node is a sink (and has more and/or stronger connections in than out) or a source (and has more and/or stronger connections out than in). This is shown schematically in Figure 31, where the number/strength of incoming directed connections is subtracted from the number/strength of outgoing directed connections. Thus, at the extreme, if the difference is negative then the node is acting as a net source, and if it is positive, the node is acting as a net sink. More generally, as can be seen in a plot such as that shown in Figure 40, lower values are indicative of more/stronger outgoing connections, and higher values are indicative of more/stronger incoming connections.

After deriving the difference between incoming and outgoing connection for all nodes, it is possible then to provide a heat map indicative of the location and intensity of sinks and sources within a patient's brain. This may include a step of defining each node as either a sink or a source. n example of such a heat map is shown in Figure 32. In this example, blue regions (arrow A) indicate more outgoing connections and so contain more source nodes whereas red/yellow regions (arrow B) indicate more incoming connections and so contain more sink nodes. This type of heat map may be referred to as a "brainprint".

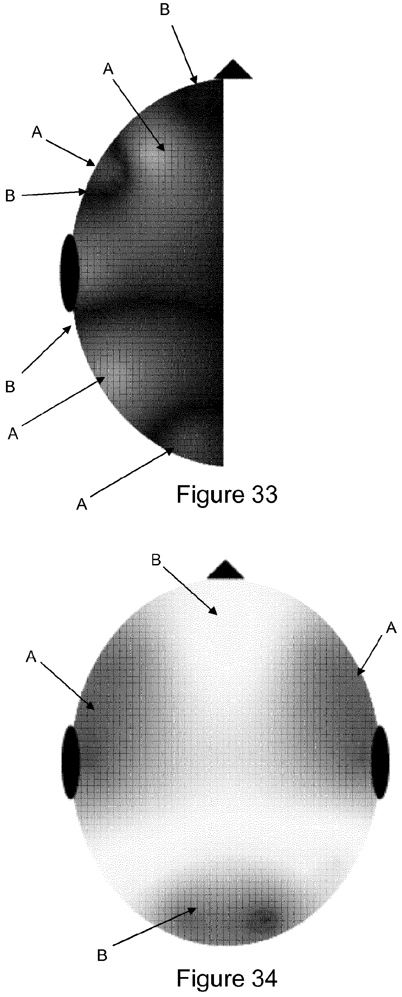

Figure 33 illustrate a visualisation of the asymmetry in the heat map of Figure 32, where the number of sources and sinks on either side are compared. A higher difference of sources and sinks between the left and right sides of the heat map appear as yellow (arrow A) whereas lower differences appear as black (arrow B).

The methods discussed above were used to analyse data provided from 329 subjects divided into 167 diagnosed subjects (DS) and 162 paired volunteers (PV) at their initial assessment (visit 1):

| | DS MEAN (SD) | PV MEAN (SD) |
|---|---|---|
| Age (at visit 1) | 70.23 (9.10) | 69.18 (11.21) |
| Gender | M: 80, F: 87 | M: 75, F: 87 |
| MMSE Visit 1 | 23.29 (2.60) | 28.80 (0.99) |
| ADAS-Cog V1 | 16.26 (7.21) | 6.04 (2.81) |
| CDR | 0.62 (0.22) | 0.06 (0.16) |

MMSE - Mini mental state examination; ADAS-Cog - Alzheimer's Disease Assessment Scale-cognitive subscale

As can be seen, diagnosed subjects are significantly more impaired cognitively on the MMSE and ADAS-Cog psychometric scales, and also have a higher score on the overall Clinical Dementia Rating (CDR) scale. Otherwise, there are no differences in age or sex distribution.

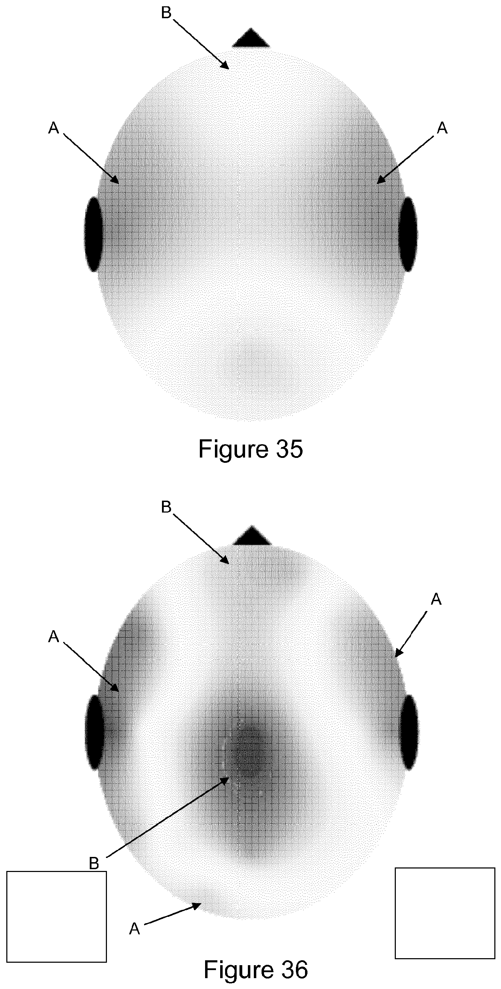

Figure 34 shows a heat map visualising the location of sinks and sources within the brain of the group of diagnosed subjects at baseline. Arrow A indicates area blue areas that contain more/stronger sources and Arrow B indicates red areas that contain more/stronger sinks. Figure 35 shows a heat map visualising the location of sinks and sources within the brain of the group of paired volunteers. When comparing the two images, it becomes clear that sufferers of AD have significantly stronger sources (i.e. more/stronger outgoing connections, shown in blue) in their frontal lobes and significantly stronger sinks (i.e. more/stronger incoming connections, shown in red/orange) in their posterior parietal, temporal and occipital lobes than the paired volunteer.

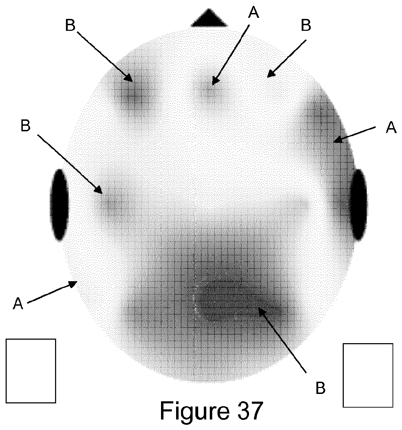

A machine learning classifier was trained on a set of the data provided by the 329 subjects discussed above. The β-band EEG data from 100 seconds of brain activity during eyes closed resting state was used in each case to prepare the rPDC network. The machine learning classifier was then used to classify all 329 subjects as either AD or paired volunteers (PV) achieving 95% accuracy. Moreover, the machine learning classifier can be used to estimate the probability that a subject has AD allowing for more than just a binary decision. For example, the subject whose heat map is shown in Figure 36 has AD. The patient was known to have AD through clinical diagnosis. The machine learning classifier estimated with a 99% probability that the patient has AD and thus correctly classified this subject. Figure 37 is a further example of a heat map from a subject known to have AD through clinical diagnosis. In this instance, the machine learning classifier estimated that there was a 63% probability that the patient had AD, and (therefore) a 37% probability that the patient did not have AD. This information can be used to determine a patient's susceptibility to AD, where no clinical diagnosis has been made. Furthermore, the specific distribution pattern of the abnormal sink regions, which are indicative of underlying dysfunction, could be correlated with specific patterns of clinical testing for further more detailed neuropsychological testing and clinical evaluation in the future. For example, the case illustrated in Figure 36 may have a form of dementia other than AD, although classified herein as suffering from AD.

Psychometric testing of the apparently healthy cohort showed downward cognitive trajectory on the Hopkins Verbal Learning Test over 18 months in a subset of the subjects. The characteristics of the cohort were as follows. As can be seen, there was no difference on cognitive score at baseline on the MMSE scale between those found to be at risk and those found not at risk of decline.

| | At Risk Mean (SD) | Not At Risk Mean (SD) |
|---|---|---|
| N | 15 | 88 |
| Age | 69.2 (5.82) | 67.43 (4.69) |
| Gender | 7 female, 8 male | 58 female, 30 male |
| MMSE | 28.5 (1.74) | 28.84 (1.32) |
| Years of education | 11.73 (1.37) | 11.64 (1.23) |

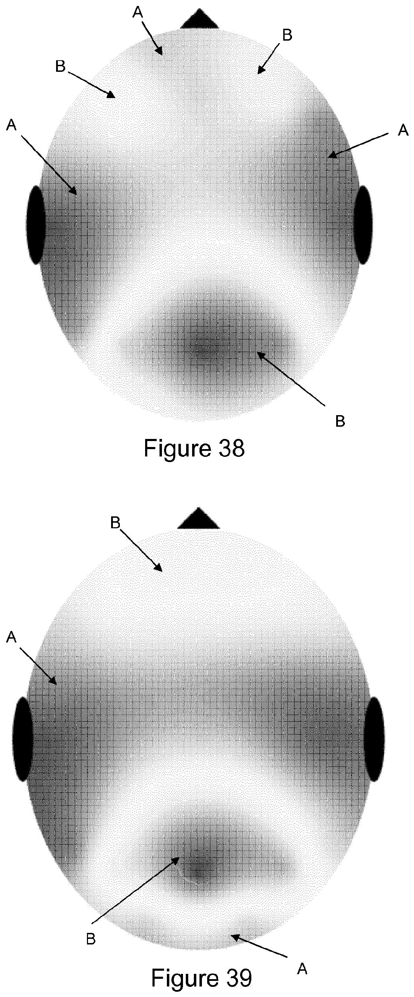

The heat map of the group of at risk subjects is shown in Figure 38, whereas the heat map of the group of not at risk subjects is shown in Figure 39. As both groups are taken from an apparently healthy cohort, the differences here are not as clear as for the AD versus PV groups above. Figure 38 shows more/stronger sinks in the posterior brain regions visible as more intense red/orange in the heat map. Figure 40 is a box and whisker plot comparing sources and sinks in EEG networks from frontal and posterior brain regions at group level. As can be seen from this figure, the at risk group is characterised by increase outgoing activity from the frontal cortex and increased incoming activity to the posterior brain regions. The EEG recordings were performed at baseline, prior to any measurable decline based on the Hopkins Verbal Learning Task. Therefore, an apparently normal subject who is at risk of decline over the following 18 months may be identified already at baseline on the basis of the heat map of their brain activity obtained non-invasively by EEG analysis.

As has been shown by the above, there are clear differences in networks between diagnosed subjects and paired volunteers. These differences are highly significant at group level. As will be appreciated, the first version of the machine learning classifier has a higher level of accuracy than routine superficial clinical assessment and gives probabilities of having AD at the individual subject level which can be used for decision making in further clinical management.

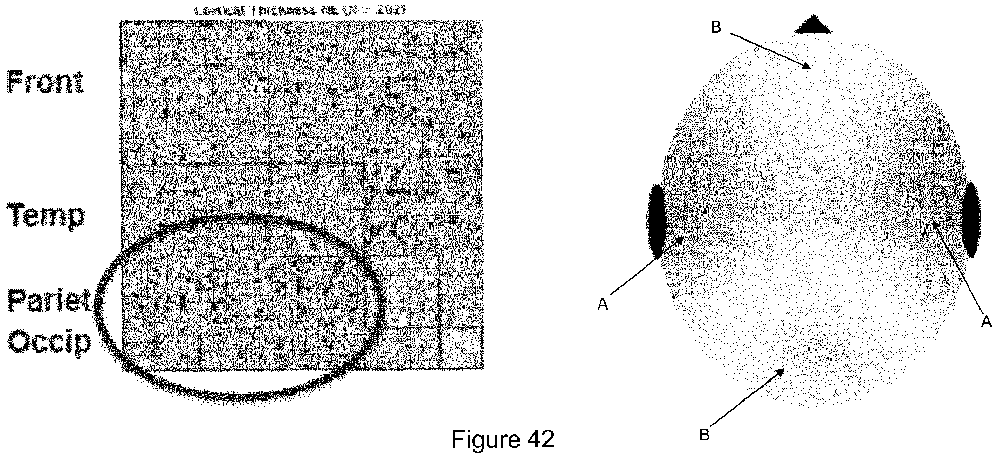

Figure 41 shows a comparison between the cortical thickness correlation matrixes (discussed above) at week 0 for the ach0 AD group as compared to the group level diagnosed subjects' heat map. As can be seen, there are a significant number of inverse non-homologous correlations between the frontal lobe and the posterior parietal and occipital brain regions. The heat map of the group of diagnosed subjects shows the same phenomenon in terms of brain connectivity as measured by EEG. Both structural and EEG approaches show the same pattern of increased frontal to posterior activity. Figure 42 shows a comparison between the cortical thickness correlation matrix (as discussed above) at week 0 for the healthy elderly group, as compared to the group level paired volunteers' heat map. As can be seen, the absence of any inverse non-homologous correlations between the frontal and posterior parietal and occipital brain regions is matched on the EEG by an absence of increased anterior to posterior electrical activity.

The increase in non-homologous inter-lobar compensatory inverse correlation networks seen in Figure 41 provides the structural basis for the characteristic heat map changes seen as the functional EEG changes.

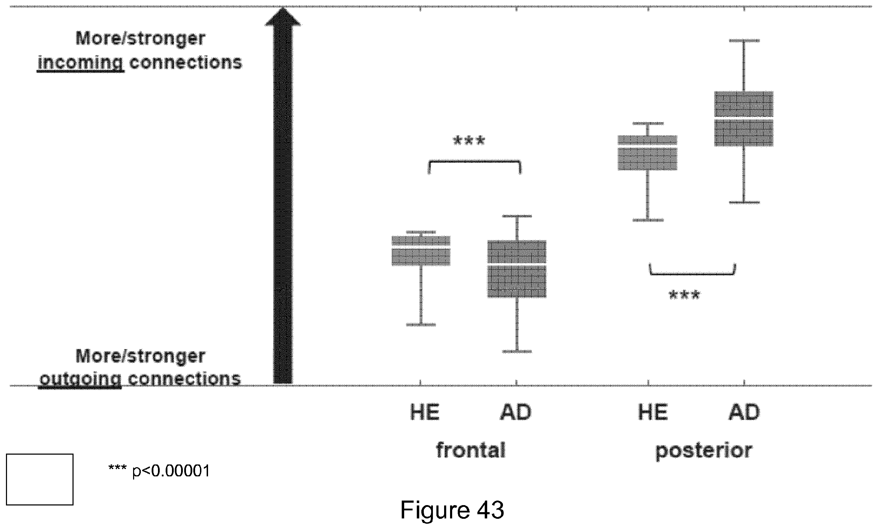

Figure 43 shows a box and whisker plot, showing quantitative differentiation of mild AD from elderly controls. As can be seen, AD subjects have more outgoing activity from the frontal cortex and more incoming activity to posterior cortex in the β-band.

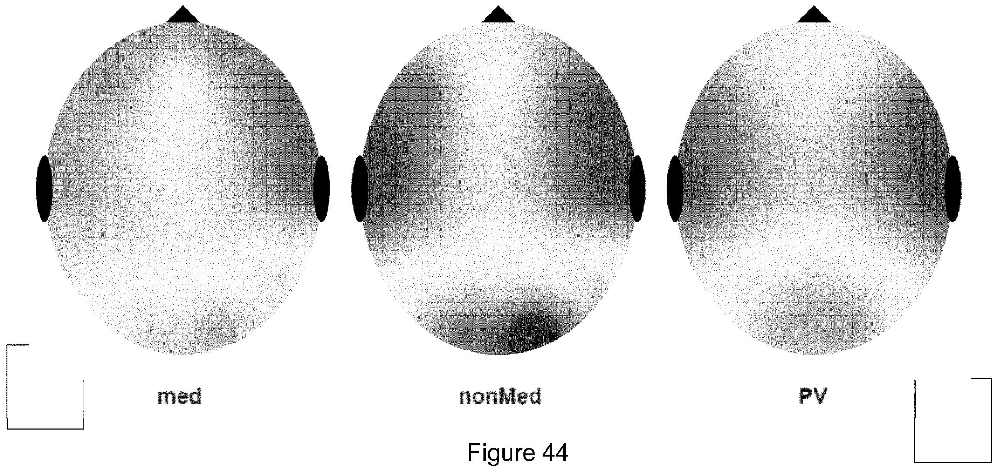

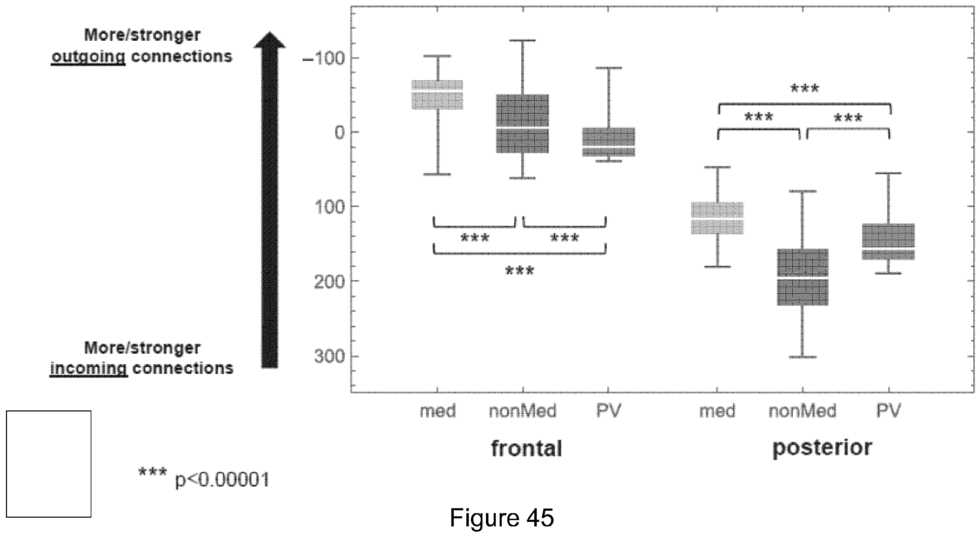

Figure 44 shows three heat maps, from left to right they are: a group of diagnosed AD subject who have been medicated with symptomatic treatments (med), a group of diagnosed AD subjects who have not been medicated with symptomatic treatments (nonMed), and a group of paired volunteers. Figure 45 is a box and whisker plot comparing the group level networks with medication, without medication, and paired volunteers. This data comes from a preliminary study comprising 53 diagnosed subjects (DS), 15 on standard medication and 38 not on standard medication. The characteristics of the two groups are shown in the table below. While the non-medicated group is substantially younger, there is no difference between the two groups in terms of cognitive score as measured by MMSE or sex distribution.

| | Med | nonMed |
|---|---|---|
| AGE | 75.2 (7.6) | 67.5 (9.5) *** |
| GENDER | 8 F: 7M | 23 F: 15 M |
| MMSE | 23.84 (1.95) (N=13) | 23.37 (2.41) |

| | | |
|---|---|---|
| *** p< 0.005 | | |

There were also no statistically significant differences in ADAS-Cog or CDR scales.

As can be seen in Figures 44 and 45, both groups of AD subjects have more outing activity from the frontal cortex in the β-band than the paired volunteers. Further, it can be seen that symptomatic treatment(s) increases activity outgoing from the frontal lobe compared to the non-medicated group. This is shown in a box and whisker plot in Figure 45. The medicated group has significantly more outgoing electrical activity from the frontal cortex. In the posterior brain regions, symptomatic treatment(s) reduces the need for supportive incoming electrical activity.

The frontal lobes show the same phenomenon by EEG as that shown by structural analyses of correlation networks in Figures 17A-D and Figures 18A-D. Figure 44 shows that the differences which can be detected at the group level by MRI structural analysis can be detected also by EEG. It should be noted that although the structural analysis of network differences between patients receiving and not receiving symptomatic treatment(s) suggests an increase in non-homologous inter-lobar connectivity directed to the posterior regions of the brain, the EEG analysis shows less incoming activity directed to the posterior regions. It is hypothesised at present that symptomatic treatment(s) increases incoming activity to the posterior brain regions in other frequency bands.

The systems and methods of the above embodiments and examples may be implemented in a computer system (in particular in computer hardware or in computer software) in addition to the structural components and user interactions described.

The term "computer system" includes the hardware, software and data storage devices for embodying a system or carrying out a method according to the above described embodiments and examples. For example, a computer system may comprise a central processing unit (CPU), input means, output means and data storage. Preferably the computer system has a monitor to provide a visual output display. The data storage may comprise RAM, disk drives or other computer readable media. The computer system may include a plurality of computing devices connected by a network and able to communicate with each other over that network.

The methods of the above embodiments and examples may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-OMs; electrical storage media such as memory, including RAM, ROM and flash memory; and hybrids and combinations of the above such as magnetic/optical storage media.

### ANNEX A

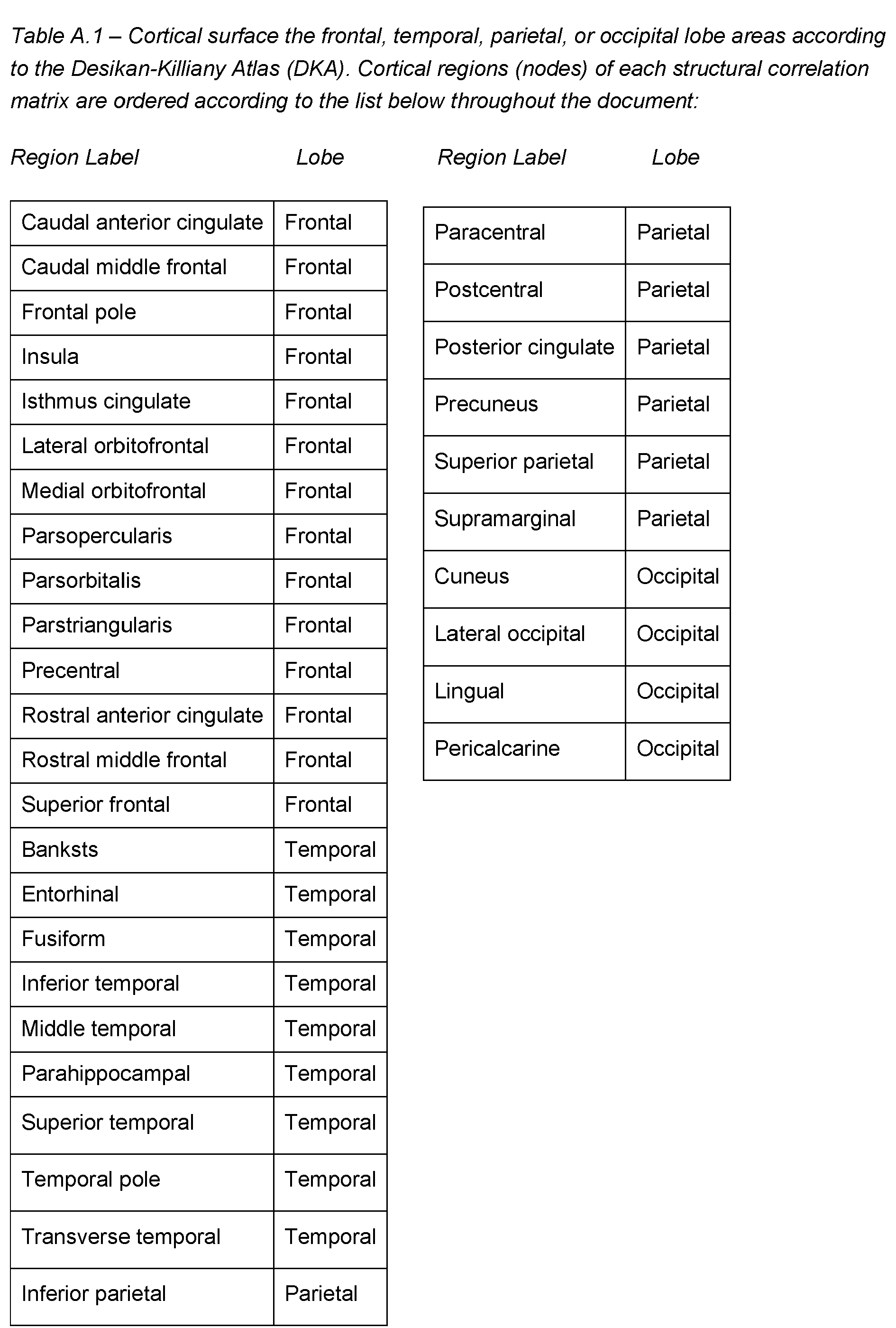

**Table A.2 - Node assignment to the frontal, temporal, parietal, or occipital lobe by the algorithm and by the DKA cortical parcellation. * nodes are wrongly assigned to a lobe for the cortical thickness network and ⁺ nodes are those incorrectly assigned for the surface area network**

| Region | CT Network | | | | SA Network | | | |
|---|---|---|---|---|---|---|---|---|
| | Label by algorithm | | Label by DKA | | Label by algorithm | | Label by DKA | |
| Banksts | T | T | T | T | T | P⁺ | T | T⁺ |
| Caudal anterior cingulate | F | F | F | F | F | F | F | F |
| Caudal middle frontal | F | F | F | F | F | F | F | F |
| Cuneus | O | O | O | O | O | O | O | O |
| Entorhinal | T | T | T | T | T | T | T | T |
| Frontal pole | F | F | F | F | F | F | F | F |
| Fusiform | T | T | T | T | T | T | T | T |
| Inferior parietal | P | P | P | P | P | P | P | P |
| Inferior temporal | T | T | T | T | T | T | T | T |
| Insula | F | F | F | F | F | F | F | F |
| Isthmus cingulate | F | F | F | F | F | F | F | F |
| Lateral occipital | O | O | O | O | O | O | O | O |
| Lateral orbitofrontal | F | F | F | F | F | F | F | F |
| Lingual | O | O | O | O | O | O | O | O |
| Medial orbito frontal | F | F | F | F | F | F | F | F |
| Middle temporal | T | T | T | T | T | T | T | T |
| Paracentral | P | P | P | P | F⁺ | F⁺ | P⁺ | P⁺ |
| Parahippocampal | T | T | T | T | T | T | T | T |
| Pars opercularis | F | F | F | F | F | F | F | F |
| Pars orbitalis | F | F | F | F | F | F | F | F |
| Pars triangularis | F | F | F | F | F | F | F | F |
| Pericalcarine | O | O | O | O | O | O | O | O |
| Postcentral | P | P | P | P | P | P | P | P |
| Posterior cingulate | P* | P* | F* | F* | P | P⁺ | P | F⁺ |
| Precentral | F* | F* | P* | P* | P | P | P | P |
| Precuneus | P | P | P | P | P | P | P | P |
| Rostral anterior cingulate | F | F | F | F | F | F | F | F |
| Rostral middle frontal | F | F | F | F | F | F | F | F |
| Superior frontal | F | F | F | F | F | F | F | F |
| Superior parietal | P | P | P | P | P | P | P | P |
| Superior temporal | T | T | T | T | T | T | T | T |
| Supramarginal | P | P | P | P | P | P | P | P |
| Temporal pole | T | T | T | T | T | T | T | T |
| Transverse temporal | T | T | T | T | T | T | T | T |

| | | | | | | | | |
|---|---|---|---|---|---|---|---|---|
| Abbreviations: F - frontal, T - temporal, P - parietal, O - occipital, L - left, R - right. | | | | | | | | |

**Table A.3 - Mean cortical thickness (CT) and total surface area (SA) averaged across four lobes in each study group**

| | Frontal | Parietal | Temporal | Occipital |
|---|---|---|---|---|
| CT (mm) mean(sd) | | | | |
| HE | 2.56(0.09) | 2.67(0.02) | 2.22(0.02) | 2.05(0.03) |
| bvFTD | 2.25(0.06) | 2.42(0.08) | 2.06(0.02) | 1.94(0.01) |
| AD | 2.31(0.08) | 2.38(0.02) | 1.96(0.01) | 1.82(0.02) |

| SA (×10⁵ mm²) mean(sd) | | | | |
|---|---|---|---|---|
| HE | 43.4(0.5) | 28.9(0.6) | 22.2(0.9) | 12.3(0.8) |
| bvFTD | 41.6(0.5) | 27.5(0.8) | 21.6(0.9) | 12.0(0.9) |
| AD | 41.6(0.5) | 27.2(0.9) | 21.5(0.9) | 12.1(0.9) |

| | | | | |
|---|---|---|---|---|
| *Abbreviations: HE - Healthy elderly, bvFTD - behavioural variant frontotemporal dementia, AD - Alzheimer's disease* | | | | |

**Table A.4 Hubs of the CT network frontal, temporal, parietal, and occipital modular organisation in HE, bvFTD, and AD. Hubs were ranked according to their between-lobes participation index (p) and within-lobe z-score (z). High p / high z scores indicate so called integrative regions (i.e., nodes interacting across all lobes) and regions with low p / high z are so called provincial hubs (i.e., nodes which interact inside its own module/lobe).**

| a) Hubs of positive sub-network | | | | | |
|---|---|---|---|---|---|
| List of high p / high z nodes | | | | | |
| HE | lobe | bvFTD | lobe | AD | lobe |
| Caudal middle frontal | F | Caudal middle frontal | F | Rostral middle frontal | F |
| Superior frontal | F | Pars opercularis | F | Inferior temporal | T |
| Superior parietal | P | Rostral middle frontal | F | Superior parietal | P |
| Pericalcarine | O | Superior frontal | F | Cuneus | O |
| | | Cuneus | O | | |
| Caudal middle frontal | F | | | Caudal middle frontal | F |
| Superior frontal | F | Pars opercularis | F | Middle temporal | T |
| Middle temporal | T | Pars triangularis | F | Superior temporal | T |
| Superior parietal | P | Rostral middle frontal | F | Superior parietal | P |
| Cuneus | O | Superior frontal | F | Cuneus | O |
| | | | | Lingual | O |

| List of low p / high z nodes | | | | | |
|---|---|---|---|---|---|
| HE | lobe | bvFTD | lobe | AD | lobe |
| Middle temporal | T | Fusiform | T | Lateral orbito frontal | F |
| Superior temporal | T | | | Rostral middle frontal | F |
| Rostral middle frontal | F | | | | |
| Inferior Temporal | T | | | | |

| b) Hubs of negative sub-network | | | | | |
|---|---|---|---|---|---|
| List of high p / high z nodes | | | | | |
| HE | lobe | bvFTD | lobe | AD | lobe |
| Rostral anterior cingulate | F | Insula | F | Caudal Middle Frontal | F |
| Middle temporal | T | Precentral | F | Insula | F |
| Transverse temporal | T | Banksts | T | Transverse temporal | T |
| | | Transverse temporal | T | | |
| Rostral middle frontal | F | Insula | F | Caudal middle frontal | F |
| Parahippocampal | T | Entorhinal | T | Lateral orbito frontal | F |
| Temporal pole | T | Temporal pole | T | Temporal pole | T |

| | | | | | |
|---|---|---|---|---|---|
| *Abbreviations: HE - healthy elderly, bvFTD - behavioural variant frontotemporal dementia, AD - Alzheimer's disease, F - frontal, T - temporal, P - Parietal, O - Occipital* | | | | | |

**Table A. 5 - Hubs of the SA network frontal, temporal, parietal, and occipital modular organisation in HE, bvFTD, and AD. Hubs were ranked according to their between-lobes participation index (p) and within-lobe z-score (z). High p / high z scores indicate so called integrative nodes (i.e., nodes interacting across all lobes) and regions with low p / high z are so called provincial hubs (i.e., nodes which interact inside its own module/lobe).**

| a) Hubs of positive sub-network | | | | | |
|---|---|---|---|---|---|
| List of high p / high z nodes | | | | | |
| HE | lobe | bvFTD | lobe | AD | lobe |
| Insula | F | Pars orbitalis | F | Insula | F |
| | | Inferior temporal | T | Lateral orbito frontal | F |
| | | Middle temporal | T | Medial orbito frontal | F |
| | | | | Pars orbitalis | F |
| | | | | Rostral anterior cingulate | F |
| | | | | Middle temporal | T |
| | | | | Fusiform | T |
| | | | | Postcentral | P |
| | | | | Precuneus | P |
| | | | | Superior parietal | P |
| | | Middle temporal | T | Lateral orbito frontal | F |
| | | Temporal pole | T | Banksts | T |
| | | Precuneus | P | Middle temporal | T |
| | | Pelicarcaline | O | Pericalcarine | O |

| List of low p / high z nodes | | | | | |
|---|---|---|---|---|---|
| HE | lobe | bvFTD | lobe | AD | lobe |
| Lateral orbito frontal | F | Pars orbitalis | F | | |
| Pars orbitalis | F | Inferior temporal | T | | |
| Inferior temporal | T | Middle temporal | T | | |
| Precuneus | P | Temporal pole | T | | |
| Pericalcarine | O | Precuneus | P | | |
| Lateral orbito frontal | F | Middle temporal | T | | |
| Precuneus | P | Pelicarcarine | O | | |
| Pelicarcarine | O | | | | |

| b) Hubs of negative sub-network | | | | | |
|---|---|---|---|---|---|
| List of high p / high z nodes | | | | | |
| HE | lobe | bvFTD | lobe | AD | lobe |
| Pars opercularis | F | Caudal anterior cingulate | F | Frontal pole | F |
| Precuneus | P | Rostral middle frontal | F | Lateral orbito frontal | F |
| | | Banksts | T | Precentral | F |
| | | Transverse temporal | T | Rostral anterior cingulate | F |
| | | Inferior parietal | P | Pars opercularis | T |
| | | Paracentral | P | Transverse temporal | T |
| Superior frontal | F | Caudal anterior cingulate | F | | |
| | | Lateral orbito frontal | F | | |
| | | Rostral middle frontal | F | | |
| | | Superior frontal | F | | |

| | | | | | |
|---|---|---|---|---|---|
| *Abbreviations: HE - healthy elderly, bvFTD - behavioural variant frontotemporal dementia, AD - Alzheimer's disease, F - frontal, T - temporal, P - Parietal, O - Occipital.* | | | | | |

**Table A.6 Hubs of the CT-SA coupling network frontal, temporal, parietal, and occipital organisation in HE, bvFTD, and AD. Regions were ranked according to their between-lobes participation index (p) and within-lobe z-score (z). High p / z scores indicate so called integrative regions (that interact across all lobes)**

| a) Hubs of positive sub-network | | | | | |
|---|---|---|---|---|---|
| List of high p / high z nodes | | | | | |
| HE | lobe | bvFTD | lobe | AD | lobe |
| Caudal middle frontal | F | Pars opercularis | F | Temporal pole | T |
| Precentral | F | Transverse temporal | T | | |
| Superior frontal | F | Supra marginal | P | | |
| Inferior parietal | P | | | | |
| Inferior parietal | P | | | | |
| Transverse temporal | T | | | | |
| Inferior parietal | P | Caudal middle frontal | F | | |
| Transverse temporal | T | Pars opercularis | F | | |
| | | Pars triangularis | F | | |
| | | Superior frontal | F | | |
| | | Post central | P | | |
| | | Supramarginal | P | | |
| | | Lateral occipital | O | | |

| | | | | | |
|---|---|---|---|---|---|
| *Abbreviations: HE - healthy elderly, bvFTD - behavioural variant frontotemporal dementia, AD - Alzheimer's disease, F - frontal, T - temporal, P - parietal, O - occipital.* | | | | | |

### References

Gauthier, S. et al. "Efficacy and safety of tau-aggregation inhibitor therapy in patients with mild or moderate Alzheimer's disease: a randomised, controlled, double-blind, parallel-arm, phase 3 trial", The Lancet 388, 2873-2884 (2016)
Wilcock, G. K. et al "Potential of low dose leuco-methylthioninium bis (hydromethanesulphonate) (Imtm) monotherapy for treatment of mild Alzheimer's disease: Cohort analysis as modified primary outcome in a phase iii clinical trial. Journal of Alzheimer's disease 61, 635-657 (2018)
Feldman, H. et al "A phase 3 trial of the tau and tdp-43 aggregation inhibitor, leuco-methylthioninium bis (hydromethanesulfonate) (Imtm), for behavioural variant frontotemporal dementia (bvFTD)" Journal of Neurochemistry 138, 255 (2016)
Murray, A. D. et al "The balance between cognitive reserve and brain imaging biomarkers of cerebrovascular and Alzheimer's diseases" Brain 134, 3687-3696 (2011)
Storey, J. D. "A direct approach to false discovery rates" Journal of the Royal Statistical Society: Series B (Statistical Methodology) 64, 479-498 (2002)
Van Wijk, B. C., Stam, C.J. & Daffertshofer, A. "Comparing brain networks of different size and connectivity density using graph theory." PLoS One 5, e13701 (2010)
Rubinov M, Sporns O. "Weight-conserving characterization of complex functional brain networks", Neuroimage, 56(4):2068-79 (2011)

## Claims

1. A computer implemented method of determining patient response to a neuropharmacological intervention comprising the steps of:
obtaining structural neurological data from a plurality of patients before neuropharmacological intervention, said structural neurological data indicative of a physical structure of a plurality of cortical regions;
wherein the physical structure is cortical thickness and/or surface area;
generating a first correlation matrix from the structural neurological data by:
assigning a plurality of structure nodes corresponding to cortical regions of the brain; and
determining pair-wise correlations between pairs of the structure nodes based at least in part on corresponding data of the structural neurological data;
obtaining further structural neurological data from the plurality of patients after neuropharmacological intervention, said further structural neurological data indicative of the physical structure of the plurality of cortical regions; and
generating a second correlation matrix from the further structural neurological data by:
determining pair-wise correlations between pairs of the structure nodes based at least in part on corresponding data of the further structural neurological data;
the method including:
comparing the first correlation matrix and the second correlation matrix, and thereby determining patient response to the neuropharmacological intervention; **characterized in that** comparing the first correlation matrix and the second correlation matrix includes comparing a number and/or density of inverse correlations in the first correlation matrix to a number and/or density of inverse correlations in the second correlation matrix; and
wherein the neuropharmacological intervention is a disease modifying pharmaceutical, and efficacy is established by reduction in number and/or density of correlations between anterior and posterior brain regions of the first correlation matrix and the second correlation matrix.

2. The method of either claim 1, wherein a p-value is determined for each pair-wise correlation, and is compared to a significance level, wherein only p-values less than the significance level are used to generate the corresponding correlation matrix.

3. The method of any preceding claim, wherein assigning the plurality of structure nodes corresponding to cortical regions of the brain further includes defining groups which contain structure nodes corresponding to homologous or non-homologous lobes.

4. The method of claim 3, wherein comparing the first correlation matrix and the second correlation matrix includes comparing the number and/or density of correlations between different groups of structure nodes.

5. The method of either claim 3 or claim 4, wherein comparing the first correlation matrix and the second correlation matrix includes comparing the number and/or density of correlations between groups of structure nodes located respectively in the frontal lobe and the parietal and occipital lobes.

6. The method of any preceding claim, wherein the patients have been diagnosed with a neurocognitive disease.

7. The method of any preceding claim, wherein the neuropharmacological intervention is a disease modifying pharmaceutical, which is optionally a tau aggregation inhibitor.

8. The method of any of claims 1 - 6, wherein the neuropharmacological intervention is a symptomatic treatment.

9. The method of any preceding claim, wherein the structural neurological data is obtained via magnetic resonance imaging.

10. A system configured to perform the method of any of claims 1 - 9, the system comprising: data acquisition means, correlation matrix generation means, and comparison means.

11. A computer program comprising executable code stored on a non-transitory storage medium which, when run on a computer, causes the computer to perform the method of any of claims 1 - 9.

## Patentansprüche

1. Computerimplementiertes Verfahren zur Bestimmung des Ansprechens eines Patienten auf eine neuropharmakologische Intervention, das die folgenden Schritte umfasst:
Erhalten von strukturellen neurologischen Daten von einer Vielzahl von Patienten vor der neuropharmakologischen Intervention, wobei die strukturellen neurologischen Daten die physische Struktur einer Vielzahl von Kortexregionen aufzeigen;
wobei die physische Struktur die Kortexdicke und/oder -oberflächengröße ist;
Erzeugen einer ersten Korrelationsmatrix aus den strukturellen neurologischen Daten durch:
Zuordnen einer Vielzahl von Strukturknoten, die Kortexregionen des Hirns entsprechen; und
Bestimmen von paarweisen Korrelationen zwischen Paaren der Strukturknoten zumindest teilweise auf Grundlage von entsprechenden Daten der strukturellen neurologischen Daten;
Erhalten von weiteren strukturellen neurologischen Daten von der Vielzahl von Patienten nach einer neuropharmakologischen Intervention, wobei die weiteren strukturellen neurologischen Daten die physikalische Struktur der Vielzahl von Kortexregionen aufzeigen; und
Erzeugen einer zweiten Korrelationsmatrix aus den weiteren strukturellen neurologischen Daten durch:
Bestimmen von paarweisen Korrelationen zwischen Paaren der Strukturknoten zumindest teilweise auf Grundlage von entsprechenden Daten der weiteren strukturellen neurologischen Daten;
wobei das Verfahren Folgendes umfasst:
Vergleichen der ersten Korrelationsmatrix und der zweiten Korrelationsmatrix und dadurch Bestimmen des Ansprechens von Patienten auf die neuropharmakologische Intervention;
**dadurch gekennzeichnet, dass** das Vergleichen der ersten Korrelationsmatrix und der zweiten Korrelationsmatrix das Vergleichen der Anzahl und/oder Dichte von inversen Korrelationen in der ersten Korrelationsmatrix mit der Anzahl und/oder Dichte von inversen Korrelationen in der zweiten Korrelationsmatrix umfasst; und
wobei die neuropharmakologische Intervention ein erkrankungsmodifzierendes Pharmazeutikum ist und die Wirksamkeit durch eine Verringerung der Anzahl und/oder Dichte von Korrelationen zwischen anterioren und posterioren Hirnregionen der ersten Korrelationsmatrix und der zweiten Korrelationsmatrix nachgewiesen wird.

2. Verfahren nach Anspruch 1, wobei ein p-Wert für jede paarweise Korrelation bestimmt wird und mit einem Signifikanzniveau verglichen wird, wobei nur p-Werte, die niedriger sind als das Signifikanzniveau, zur Erzeugung der entsprechenden Korrelationsmatrix verwendet werden.

3. Verfahren nach einem der vorangegangenen Ansprüche, wobei das Zuordnen der Vielzahl von Strukturknoten, die Kortexregionen des Hirns entsprechen, weiters das Definieren von Gruppen umfasst, die Strukturknoten enthalten, die homologen oder nichthomologen Lappen entsprechen.

4. Verfahren nach Anspruch 3, wobei das Vergleichen der ersten Korrelationsmatrix und der zweiten Korrelationsmatrix das Vergleichen der Anzahl und/oder Dichte von Korrelationen zwischen verschiedenen Gruppen von Strukturknoten umfasst.

5. Verfahren nach Anspruch 3 oder Anspruch 4, wobei das Vergleichen der ersten Korrelationsmatrix und der zweiten Korrelationsmatrix das Vergleichen der Anzahl und/oder Dichte von Korrelationen zwischen Gruppen von Strukturknoten umfasst, die sich im Frontallappen, Parietallappen bzw. Okzipitallappen befinden.

6. Verfahren nach einem der vorangegangenen Ansprüche, wobei bei den Patienten eine neurokognitive Erkrankung diagnostiziert wurde.

7. Verfahren nach einem der vorangegangenen Ansprüche, wobei die neuropharmakologische Intervention ein erkrankungsmodifzierendes Pharmazeutikum ist, das gegebenenfalls ein Tau-Aggregationshemmer ist.

8. Verfahren nach einem der Ansprüche 1 bis 6, wobei die neuropharmakologische Intervention eine symptomatische Behandlung ist.

9. Verfahren nach einem der vorangegangenen Ansprüche, wobei die strukturellen neurologischen Daten durch Magnetresonanztomographie erhalten werden.

10. System, das dazu ausgelegt ist, ein Verfahren nach einem der Ansprüche 1 bis 9 auszuführen, wobei das System Folgendes umfasst: Datenerfassungsmittel, Korrelationsmatrixerzeugungsmittel und Vergleichsmittel.

11. Computerprogramm, das einen ausführbaren Code umfasst, der auf einem nichtflüchtigen Speichermedium gespeichert ist und, wenn er auf einem Computer laufen gelassen wird, veranlasst, dass der Computer ein Verfahren nach einem der Ansprüche 1 bis 9 ausführt.

## Revendications

1. Procédé mis en œuvre par ordinateur pour déterminer la réponse d'un patient à une intervention neuropharmacologique, comprenant les étapes consistant à :
obtenir des données neurologiques structurelles à partir d'une pluralité de patients avant une intervention neuropharmacologique, lesdites données neurologiques structurelles indiquant une structure physique d'une pluralité de régions corticales ;
dans lequel la structure physique est une épaisseur corticale et/ou une surface spécifique ;
générer une première matrice de corrélations à partir des données neurologiques structurelles par :
attribution d'une pluralité de nœuds de structure correspondant à des régions corticales du cerveau ; et
détermination de corrélations par paires entre des paires des nœuds de structure sur la base au moins en partie de données correspondantes des données neurologiques structurelles ;
obtenir des données neurologiques structurelles supplémentaires à partir de la pluralité de patients après une intervention neuropharmacologique, lesdites données neurologiques structurelles supplémentaires indiquant la structure physique de la pluralité de régions corticales ; et
générer une seconde matrice de corrélations à partir des données neurologiques structurelles supplémentaires par :
détermination de corrélations par paires entre des paires des nœuds de structure sur la base au moins en partie de données correspondantes des données neurologiques structurelles supplémentaires ;
le procédé incluant les étapes consistant à :
comparer la première matrice de corrélations et la seconde matrice de corrélations, et déterminer ainsi la réponse du patient à l'intervention neuropharmacologique ;
**caractérisé en ce que**
la comparaison de la première matrice de corrélations et la seconde matrice de corrélations inclut une comparaison d'un nombre et/ou d'une densité de corrélations inverses dans la première matrice de corrélations à un nombre et/ou une densité de corrélations inverses dans la seconde matrice de corrélations ; et
dans lequel l'intervention neuropharmacologique est un produit pharmaceutique modifiant la maladie, et l'efficacité est établie par réduction du nombre et/ou de la densité des corrélations entre les régions cérébrales antérieure et postérieure de la première matrice de corrélations et de la seconde matrice de corrélations.

2. Procédé selon la revendication 1, dans lequel une valeur p est déterminée pour chaque corrélation par paires, et est comparée à un niveau de signification, dans lequel seules des valeurs p inférieures au niveau de signification sont utilisées pour générer la matrice de corrélations correspondante.

3. Procédé selon l'une quelconque des revendications précédentes, dans lequel l'attribution de la pluralité de nœuds de structure correspondant à des régions corticales du cerveau inclut en outre une définition de groupes qui contiennent des nœuds de structure correspondant à des lobes homologues ou non homologues.

4. Procédé selon la revendication 3, dans lequel la comparaison de la première matrice de corrélations et de la seconde matrice de corrélations inclut une comparaison du nombre et/ou de la densité de corrélations entre différents groupes de nœuds de structure.

5. Procédé selon la revendication 3 ou la revendication 4, dans lequel la comparaison de la première matrice de corrélations et de la seconde matrice de corrélations inclut une comparaison du nombre et/ou de la densité de corrélations entre des groupes de nœuds de structure situés respectivement dans le lobe frontal et les lobes pariétal et occipital.

6. Procédé selon l'une quelconque des revendications précédentes, dans lequel les patients ont reçu un diagnostic de maladie neurocognitive.

7. Procédé selon l'une quelconque des revendications précédentes, dans lequel l'intervention neuropharmacologique est un produit pharmaceutique modifiant la maladie, qui est facultativement un inhibiteur d'agrégation de tau.

8. Procédé selon l'une quelconque des revendications 1 à 6, dans lequel l'intervention neuropharmacologique est un traitement symptomatique.

9. Procédé selon l'une quelconque des revendications précédentes, dans lequel les données neurologiques structurelles sont obtenues par imagerie par résonance magnétique.

10. Système configuré pour exécuter le procédé selon l'une quelconque des revendications 1 à 9, le système comprenant : des moyens d'acquisition de données, des moyens de génération de matrice de corrélations et des moyens de comparaison.

11. Programme informatique comprenant un code exécutable stocké sur un support de stockage non transitoire qui, lorsqu'il est exécuté sur un ordinateur, amène l'ordinateur à exécuter le procédé selon l'une quelconque des revendications 1 à 9.
